(19) Europäisches Patentamt
European Patent Office
Office européen des brevets

(11) EP 2 231 283 B1

(12) # FASCICULE DE BREVET EUROPEEN

(45) Date de publication et mention de la délivrance du brevet:
**12.09.2012 Bulletin 2012/37**

(21) Numéro de dépôt: **08865749.9**

(22) Date de dépôt: **05.12.2008**

(51) Int Cl.:
***A61Q 1/02*** (2006.01) ***A61Q 1/12*** (2006.01)
***A61K 8/06*** (2006.01) ***A61K 8/19*** (2006.01)
***A61K 8/25*** (2006.01) ***A61K 8/26*** (2006.01)
***A61K 8/891*** (2006.01) ***A61K 8/892*** (2006.01)
***A61K 8/898*** (2006.01)

(86) Numéro de dépôt international:
**PCT/FR2008/052235**

(87) Numéro de publication internationale:
**WO 2009/080965 (02.07.2009 Gazette 2009/27)**

(54) **PROCEDE COSMETIQUE UTILISANT UNE COMPOSITION COMPRENANT UNE RESINE DE SILOXANE ET UNE CHARGE MINERALE**

KOSMETISCHES VERFAHREN, BEI DEM EINE ZUSAMMENSETZUNG AUS EINEM SILOXANHARZ UND EINEM MINERALISCHEN FÜLLSTOFF VERWENDET WIRD

COSMETIC METHOD USING A COMPOSITION COMPRISING A SILOXANE RESIN AND A MINERAL FILLER

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **05.12.2007 US 992357 P**

(43) Date de publication de la demande:
**29.09.2010 Bulletin 2010/39**

(73) Titulaire: **L'Oréal**
**75008 Paris (FR)**

(72) Inventeur: **DOP, Florence**
**F-91190 Villiers Le Bacle (FR)**

(74) Mandataire: **Tezier Herman, Béatrice et al**
**Cabinet Becker & Associés**
**25, rue Louis Le Grand**
**75002 Paris (FR)**

(56) Documents cités:
**WO-A-2005/075542** **WO-A-2005/075567**
**WO-A-2009/071662** **US-A1- 2006 292 096**

- **VIRGINIE CAPRASSE ET AL: "A new silicone resin for personal care applications" RESEARCH DISCLOSURE, MASON PUBLICATIONS, HAMPSHIRE, GB, vol. 486, no. 8, 1 octobre 2004 (2004-10-01), XP007134333 ISSN: 0374-4353**
- **DIPL ING KATRIN STEINBACH AND MICHAELA GERSTACKER ET AL: "Verwendung von IR-reflektierenden Pigmenten in kosmetischen Applikationen" RESEARCH DISCLOSURE, MASON PUBLICATIONS, HAMPSHIRE, GB, vol. 523, no. 5, 1 novembre 2007 (2007-11-01), page 1086, XP007137714 ISSN: 0374-4353**
- **VERONIQUE KOWANDY ET AL: "Bodied MQ-T Propyl Silicone Resins in Color Cosmetic Applications" IP.COM JOURNAL, IP.COM INC., WEST HENRIETTA, NY, US, 4 décembre 2008 (2008-12-04), XP013127272 ISSN: 1533-0001**
- **ZHIKAI KEVIN FANG ET AL: "New formulation capabilities with three new silicone resin flake products" IP.COM JOURNAL, IP.COM INC., WEST HENRIETTA, NY, US, 4 décembre 2008 (2008-12-04), XP013128295 ISSN: 1533-0001**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 231 283 B1

## Description

L'invention concerne un procédé cosmétique de maquillage et/ou de soin de la peau, comprenant l'application sur la peau d'une composition cosmétique comprenant une résine de siloxane et une charge minérale choisie parmi le talc, le mica, la silice, le kaolin, le nitrure de bore, le carbonate de calcium précipité, le carbonate et l'hydro-carbonate de magnésium, l'hydroxyapatite, les microsphères de silice creuses, les microcapsules de verre ou de céramique, l'argile, le quartz, la poudre de diamant naturel et leur mélange. L'invention concerne en particulier des compositions de soin ou de maquillage de la peau.

Il est connu d'utiliser des produits de maquillage afin d'apporter de la couleur et de la matité sur la peau. Il est attendu de ces produits de maquillage que le résultat maquillage tienne au cours de la journée.
Un produit de maquillage doit aussi être confortable. Par confortable, on entend un produit glissant sur la peau, sans sensation de frottement lorsque l'on applique le produit sur la peau, mais également un produit conférant un touché doux sur ladite peau.

Il est connu de l'homme de l'art de conférer les propriétés de confort à un produit de maquillage, comme cela est décrit ci-dessus, en formulant ces produits avec des charges.
Il est également connu de l'homme de l'art de conférer les propriétés de tenue du maquillage en formulant ces produits avec des résines siliconées, des polyacrylates, ou encore des latex, qui ont toutefois tendance à procurer une sensation d'inconfort après application, avec un touché collant.

Le but de la présente invention est donc de proposer un procédé de maquillage permettant d'obtenir des propriétés de tenue du maquillage satisfaisantes, tout en conservant le confort durant et après application.

Ce but, ainsi que d'autres, sont atteints par la présente invention qui décrit notamment un procédé cosmétique de maquillage et/ou de soin de la peau comprenant l'application sur la peau d'une composition comprenant, dans un milieu physiologiquement acceptable :

- i) une résine de siloxane comprenant les unités :

  (i) $(R^1{}_3SiO_{1/2})a$
  (ii) $(R^2{}_2SiO_{2/2})b$
  (iii) $(R^3SiO_{3/2})c$ et
  (iv) $(SiO_{4/2})d$

  avec

  $R^1$, $R^2$ et $R^3$ représentant indépendamment un groupement alkyle ayant de 1 à 8 atomes de carbone, un groupement aryle, un groupement carbinol ou un groupement amino,
  a étant compris entre 0,05 et 0,5,
  b étant compris entre zéro et 0,3,
  c étant supérieur à zéro,
  d étant compris entre 0,05 et 0,6,
  a+b+c+d=1,

  à condition que plus de 40 % en moles des groupements $R^3$ de la résine de siloxane soient des groupements propyle, et
- ii) au moins une charge minérale choisie parmi le talc, le mica, la silice, le kaolin, le nitrure de bore, le carbonate de calcium précipité, le carbonate et l'hydro-carbonate de magnésium, l'hydroxyapatite, les microsphères de silice creuses, les microcapsules de verre ou de céramique, l'argile, le quartz, la poudre de diamant naturel et leur mélange.

L'association utilisée selon l'invention permet d'obtenir un fini doux sur la peau et des propriétés cosmétiques stables dans le temps, en particulier tout au long de la journée. Le produit est également glissant sur la peau, lors de l'application, sans sensation de frottement, et confère un toucher doux sur ladite peau.

La composition selon l'invention peut se présenter sous diverses formes, notamment sous forme de poudres (libres ou compactes), de dispersion anhydre, émulsion eau/huile ou eau/cire, huile/eau, multiples (comme une émulsion eau/huile/eau ou huile/eau/huile) ou cire/eau, ou encore sous forme de gel. De préférence, la composition selon l'invention se présente sous la forme de poudres (libres ou compactes), de dispersion anhydre ou d'émulsions, de préférence d'émulsion inverse (i.e., émulsion eau/huile).

**RESINES DE SILOXANE**

Les résines de siloxane utilisables selon l'invention peuvent être obtenues par un procédé comprenant la réaction de :

A) une résine MQ comprenant au moins 80 % en moles d'unités $(R^1{}_3SiO_{1/2})_a$ et $(SiO_{4/2})_d$

$R^1$ représentant un groupement alkyle ayant de 1 à 8 atomes de carbone, un groupement aryle, un groupement carbinol ou un groupement amino, a et d étant supérieurs à zéro, le rapport a/d étant compris entre 0,5 et 1,5 ;

et de
B) une résine de propyle T comprenant au moins 80 % en moles d'unités $(R^3SiO_{3/2})c$,

$R^3$ représentant un groupement alkyle ayant de 1 à 8 atomes de carbone, un groupement aryle, un groupement carbinol ou un groupement amino, c étant supérieur à zéro,
à condition qu'au moins 40 % en moles des groupements $R^3$ soient des groupements propyle,

où le ratio massique A/B est compris entre 95:5 et 15:85.
De préférence, le rapport A/B est inférieur ou égal à 70:30. De manière avantageuse, le rapport A/B est égal à 30:70 ou 50:50.
Les résines utilisables selon l'invention sont notamment celles décrites dans la demande WO 2005/075542.
La résine MQ-T propyle selon l'invention comprend des unités :

(i) $(R^13SiO_{1/2})_a$
(ii) $(R^2{}_2SiO_{2/2})_b$
(iii) $(R^3SiO_{3/2})_c$ et
(iv) $(SiO_{4/2})_d$

qui sont connues de l'art antérieur et qui correspondent respectivement aux unités M, D, T et Q.
La quantité de chaque unité présente dans la résine MQ-T propyle peut être exprimée en fraction molaire (ie a, b, c ou d) du nombre total de moles de toutes les unités M, D, T et Q présentes dans la résine MQ-T propyle.
La valeur de a (fraction molaire d'unités M) est comprise entre 0,05 et 0,5, ou alternativement entre 0,15 et 0,4.
La valeur de b (fraction molaire d'unités D) est comprise entre 0 et 0,3, ou alternativement entre 0 et 0,1, ou alternativement entre 0 et 0,05. Ainsi, la résine MQ-T propyle selon l'invention peut être exempte d'unité D, ou alternativement peut comprendre jusqu'à 0.3 fraction molaire d'unités D.
De préférence, la résine MQ-T propyle selon l'invention est exempte d'unité D.
La valeur de c (fraction molaire d'unités T) est supérieure à 0, ou alternativement comprise entre 0,05 et 0,65, ou alternativement comprise entre 0,4 et 0,65.
La valeur de d (fraction molaire d'unités Q) est comprise entre 0,05 et 0,6, ou alternativement entre 0,2 et 0,6, ou alternativement comprise entre 0,2 et 0,55.
La résine MQ-T propyle selon l'invention est caractérisée par le fait qu'au moins 40% en moles, de préférence au moins 50% en moles, de préférence au moins 90% en moles de groupes alkyles $R_3$ des unités T sont des groupes propyles.
Les radicaux $R^1$, $R^2$, $R^3$ des unités de la résine MQ-T propyle représentent indépendamment un groupement alkyle ayant de 1 à 8 atomes de carbone, un groupement aryle, un groupement carbinol ou un groupement amino.
Les groupements alkyle peuvent notamment être choisis parmi les groupements méthyle, éthyle, propyle, butyle, pentyle, hexyle et octyle. De préférence, le groupe alkyle est un groupe méthyle ou un groupe propyle.
Les groupements aryle peuvent être choisis parmi les groupements phényle, naphthyle, benzyle, tolyle, xylyle, xényle, méthylphényle, 2-phényléthyle, 2-phényl-2-méthyléthyle, chlorophényle, bromophényle et fluorophényle, le groupement aryle étant préférentiellement un groupement phényle.
Dans la présente invention, par « groupement carbinol », on entend tout groupement contenant au moins un radical hydroxyle lié à un carbone (COH). Les groupements carbinol peuvent ainsi contenir plus d'un radical COH, tel que par exemple

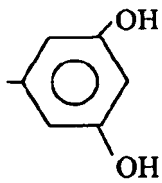

.

Si le groupement carbinol est exempt de groupements aryle, il comporte au moins 3 atomes de carbone. Si le groupement carbinol comprend au moins un groupement aryle, il comporte au moins 6 atomes de carbone.

Comme exemples de groupement carbinol exempt de groupements aryle comportant au moins 3 atomes de carbone, on peut citer les groupements de formule $R^4OH$ dans laquelle $R^4$ représente un radical hydrocarboné bivalent comportant au moins 3 atomes de carbone ou un radical hydrocarbonoxy bivalent comportant au moins 3 atomes de carbone. Comme exemples de groupement $R^4$, on peut citer des radicaux alkylène tels que $-(CH_2)_x-$, la valeur de x étant comprise entre 3 et 10, $-CH_2CH(CH_3)-$, $-CH_2CH(CH_3)CH_2-$, $-CH_2CH_2CH(CH_2CH_3)CH_2CH_2CH_2-$ et $-OCH(CH_3)(CH_2)_x-$, la valeur de x étant comprise entre 1 et 10.

Comme exemples de groupement carbinol comportant des groupements aryle présentant au moins 6 atomes de carbone, on peut citer les groupements de formule $R^5OH$ dans laquelle $R^5$ représente un radical arylène tel que $-(CH_2)_xC_6H_4-$, x ayant une valeur comprise entre 0 et 10, $-CH_2CH(CH_3)(CH_2)_xC_6H_4-$, x ayant une valeur comprise entre 0 et 10, $-(CH_2)xC_6H_4(CH_2)_x-$, x ayant une valeur comprise entre 1 et 10. Les groupements carbinol comportant des groupements aryle comportent généralement de 6 à 14 atomes.

Par groupement amino selon l'invention, on entend notamment des groupements de formule $-R^6NH_2$ ou $-R^6NHR^7NH_2$, $R^6$ représentant un radical hydrocarboné bivalent ayant au moins 2 atomes de carbone et $R^7$ représentant un radical hydrocarboné bivalent ayant au moins 2 atomes de carbone. Le groupement $R^6$ représente généralement un radical alkylène ayant de 2 à 20 atomes de carbone. Comme exemples de groupement $R^6$, on peut citer les groupements éthylène, propylène, $-CH_2CHCH_3-$, butylène, $-CH_2CH(CH_3)CH_2-$, pentaméthylène, hexaméthylène, 3-éthyl-hexaméthylène, octaméthylène et décaméthylène.

Le groupement $R^7$ représente généralement un radical alkylène ayant de 2 à 20 atomes de carbone. Comme exemples de groupement $R^7$, on peut citer les groupements éthylène, propylène, $-CH_2CHCH_3-$, butylène, $-CH_2CH(CH_3)CH_2-$, pentaméthylène, hexaméthylène, 3-éthyl-hexaméthylène, octaméthylène et décaméthylène.

Les groupements amino sont généralement $-CH_2CH_2CH_2NH_2$ et $-CH_2(CH_3)CHCH_2(H)NCH_3$, $-CH_2CH_2NHCH_2CH_2NH_2$, $-CH_2CH_2NH_2$, $-CH_2CH_2NHCH_3$, $-CH_2CH_2CH_2CH_2NH_2$, $-(CH_2CH_2NH)_3H$ et $-CH_2CH_2NHCH_2CH_2NHC_4H_9$.

De préférence, $R^1$ représente un groupe méthyle, $R^2$ représente un groupe méthyle ou un groupe phényle, et $R^3$ représente un groupe propyle.

De préférence, la résine MQ-T propyle selon l'invention est exempte d'unité D, et $R^1$ représente un groupe méthyle, et $R^3$ représente un groupe propyle.

Les unités siloxane D, T ou Q de la résine MQ-T propyle selon l'invention peuvent comprendre des groupes hydroxy (-OH) et/ou des groupes alcoxy. De telles unités siloxane comprenant des groupes hydroxy et/ou alkoxy sont présentes communément dans des résines siloxane ayant comme formule générale $R_nSiO_{(4-n)/2}$.

Ces groupes hydroxy résultent typiquement de la réaction d'un groupe hydrolysable sur l'unité siloxane avec l'eau ; les groupes alkoxy résultent d'une hydrolyse incomplète quand des précurseurs alkoxysilanes sont utilisés ou résultent de l'échange d'alcool avec des groupes hydrolysables.

De préférence la quantité totale en poids de groupements -OH présente dans la résine MQ-T propyle est d'environ 3%, de préférence 2%, de préférence 1,5%. De préférence, la quantité totale en poids de groupes alcoxy présente dans la résine MQ-T propyle est inférieure ou égale à 20% en poids, de préférence inférieure ou égale à 10% en poids.

De préférence, la résine de siloxane présente dans ladite composition comprend les unités :

(i) $(R^1_3SiO_{1/2})a$
(iii) $(R^3SiO_{3/2})c$ et
(iv) $(SiO_{4/2})d$

avec

$R^1$ et $R^3$ représentant indépendamment un groupement alkyle ayant de 1 à 8 atomes de carbone, de préférence $R^1$ est un groupe méthyle et $R^3$ est un groupe propyle,
a étant compris entre 0,05 et 0,5,
c étant supérieur à zéro,
d étant compris entre 0,05 et 0,6,

a+c+d=1,

à condition que plus de 40 % en moles des groupements $R^3$ de la résine de siloxane soient des groupements propyle.

Il n'existe pas de restrictions relatives à la masse moléculaire des résines de propyle siloxane MQ-T, mais généralement la masse moléculaire moyenne en nombre ($M_N$) est comprise entre 3 000 et 10 000, ou encore entre 5 000 et 8 000.

Les résines MQ-T propyle utilisables selon l'invention peuvent être préparées selon les procédés connus dans l'état de la technique pour préparer des résines de siloxane de formule générale $R_nSiO_{(4-n)/2}$ où R est un groupe alkyle et n est inférieur à 1,8. Alternativement, les résines MQ-T propyle peuvent être préparées selon les méthodes décrites ci-dessous.

Les résines MQ-T propyle selon l'invention sont illustrées par les résines MQ-T propyle comprenant les unités suivantes :

$((CH_3)_3SiO_{1/2})_a$
$(R^3SiO_{3/2})_c$ où $R^3 = CH_3CH_2CH_2$-, et
$(SiO_{4/2})_d$ ;

ou les unités suivantes :

$((CH_3)_3SiO_{1/2})_a$
$((CH_3)_2SiO_{2/2})_b$
$(R^3SiO_{3/2})_c$ où $R^3 = CH_3CH_2CH_2$-, et
$(SiO_{4/2})_d$ ;

ou les unités suivantes :

$((CH_3)_3SiO_{1/2})_a$
$((CH_3)_2SiO_{2/2})_b$, $((CH_3)(C_6H_5)SiO_{2/2})_b$
$(R^3SiO_{3/2})_c$ où $R^3 = CH_3CH_2CH_2$-, et
$(SiO_{4/2})_d$ ;

ou les unités suivantes :

$((CH_3)_3SiO_{1/2})_a$
$((CH_3)_2SiO_{2/2})_b$
$(R^3SiO_{3/2})_c$ où $R^3 = CH_3CH_2CH_2$-, et $(C_6H_5SiO_{3/2})_c$
$(SiO_{4/2})_d$ ;

ou les unités suivantes :

$((CH_3)_3SiO_{1/2})_a$
$((CH_3)_2SiO_{2/2})_b$, $((CH_3)(C_6H_5)SiO_{2/2})_b$
$(R^3SiO_{3/2})$où $R^3 = CH_3CH_2CH_2$-, et $(C_6H_5SiO_{3/2})_c$
$(SiO_{4/2})_d$ ;

où a a une valeur totale dans la résine comprise entre 0,05 et 0,5, la somme b+b' a une valeur totale dans la résine comprise entre 0 et 0,3, c a une valeur totale dans la résine comprise entre 0,05 et 0,65, et d a une valeur totale dans la résine comprise entre 0,05 et 0,6.

Les résines de siloxane utilisables selon l'invention peuvent être obtenues par un procédé comprenant la réaction entre :

A) une résine MQ comprenant au moins 80 % en moles d'unités $(R^1{}_3SiO_{1/2})_a$ et $(SiO_{4/2})_d$

$R^1$ représentant un groupement alkyle ayant de 1 à 8 atomes de carbone, un groupement aryle, un groupement carbinol ou un groupement amino, a et d étant supérieurs à zéro,
le rapport a/d étant compris entre 0,5 et 1,5 ;

et

B) une résine de T propyle comprenant au moins 80 % en moles d'unités $(R^3SiO_{3/2})c$ O,

$R^3$ représentant un groupement alkyle ayant de 1 à 8 atomes de carbone, un groupement aryle, un groupement carbinol ou un groupement amino,
c étant supérieur à zéro,
à condition qu'au moins 40 % en moles des groupements $R^3$ soient des groupements propyle,

où le ratio massique A/B est compris entre 95:5 et 15:85.

La composant A) est une résine MQ comprenant au moins 80 % en moles d'unités $(R^1{}_3SiO_{1/2})_a$ et $(SiO_{4/2})_d$ où $R^1$ est tel que défini ci-dessus, ie représente un groupement alkyle ayant de 1 à 8 atomes de carbone, un groupement aryle, un groupement carbinol ou un groupement amino, a et d étant supérieurs à zéro, et le rapport a/d étant compris entre 0,5 et 1,5.
Les résines MQ utilisables comme composant A), et leur méthode de préparation, sont connues de l'art antérieur. Par exemple, le brevet US 2 814 601, appartenant à Currie et al., daté du 26 novembre 1957 décrit un procédé de fabrication de résines MQ par transformation d'un silicate hydrosoluble en un monomère d'acide silicique ou un oligomère d'acide silicique en utilisant un acide. Une fois la polymérisation adéquate réalisée, des extrémités triméthylchlorosilane sont introduites pour obtenir la résine MQ. Un autre procédé de préparation de résines MQ est décrit dans le brevet US 2 857 356 appartenant à Goodwin, daté du 21 octobre 1958. Goodwin décrit un procédé de fabrication d'une résine MQ par cohydrolyse d'un mélange d'un silicate d'alkyle et d'un organopolysiloxane trialkylsilane hydrolysable avec de l'eau.
Les résines MQ convenant en tant que composant A) dans la présente invention peuvent contenir des unités D et T, à condition d'au moins 80 % en moles, voire 90 % en moles des unités de siloxane totales soient des unités M et Q. Les résines MQ peuvent également contenir des groupements hydroxy. Les résines MQ peuvent ainsi comprendre des groupes hydroxy en quantité totale en poids comprise entre 2 et 10%, de préférence entre 2 et 5%. Les résines MQ peuvent également comporter des extrémités supplémentaires, des groupements hydroxy résiduels étant pour cela mis en réaction avec les groupements M.

Le composant B) est une résine de T propyle comprenant au moins 80 % en moles d'unités $(R^3SiO_{3/2})c$, $R^3$ étant tel que défini ci-dessus, ie représentant un groupement alkyle ayant de 1 à 8 atomes de carbone, un groupement aryle, un groupement carbinol ou un groupement amino, c étant supérieur à 0, à condition qu'au moins 40 % en moles des groupements $R^3$ soient des groupements propyle. De préférence, la résine T propyle selon l'invention est une résine de silsesquioxane. Les résines de silsesquioxane sont bien connues dans l'état de la technique et sont généralement obtenues par hydrolyse d'un organosilane comportant trois groupements hydrolysables, tels que des groupements halogène ou alcoxy, présents dans la molécule. Le composant B) peut ainsi être obtenu par hydrolyse de propyltriméthoxysilane, propyltriéthoxysilane, propyltripropoxysilane, ou par cohydrolyse des propylalcoxysilanes susmentionnés avec divers alcoxysilanes. Comme exemples de ces alcoxysilanes, on peut citer le méthyltriméthoxysilane, le méthyltriéthoxysilane, le méthyltriisopropoxysilane, le diméthyldiméthoxysilane et le phényltriméthoxysilane. Le propyltrichlorosilane peut également être hydrolysé seul, ou en présence d'alcool. Dans ce cas, la cohydrolyse peut être réalisée en ajoutant du méthyltrichlorosilane, du diméthyldichlorosilane, du phényltrichlorosilane ou des chlorosilanes similaires et du méthyltriméthoxysilane, du méthyltriéthoxysilane, du méthyltriisopropoxysilane ou des méthylalcoxysilanes similaires. Comme alcools convenant en ce but, on peut citer le méthanol, l'éthanol, l'alcool n-propylique, l'alcool isopropylique, le butanol, le méthoxy éthanol, l'éthoxy éthanol ou des alcools similaires. Comme exemples de solvants de type hydrocarbures pouvant être utilisés, on peut citer le toluène, le xylène ou des hydrocarbures aromatiques similaires ; l'hexane, l'heptane, l'isooctane ou des hydrocarbures saturés linéaires ou en partie ramifiés similaires ; ainsi que le cyclohexane ou des hydrocarbures aliphatiques similaires.

Les résines de T propyle comme composant B) selon l'invention peuvent contenir des unités M, D et Q, à condition qu'au moins 80 % en moles, voire 90 % en moles des unités de siloxane totales soient des unités T. Les résines de T propyle peuvent également contenir des groupements hydroxy. De préférence, les résines de T propyle comprennent entre 3 et 8% en poids de groupements hydroxy.

Un polyorganosiloxane peut également être ajouté au procédé selon l'invention en tant que composant C). Les polyorganosiloxanes utiles comme composant C) selon l'invention comprennent des unités $R^2{}_2SiO_{2/2}$, ou $R^3SiO_{3/2}$. Le polyorganosiloxane peut être ajouté pour introduire différentes unités D et T dans les résines MQ-T propyle, afin de modifier les propriétés des résines résultantes. La structure ou la formule du polyorganosiloxane n'est pas limitative, à condition que ledit polyorganosiloxane comprenne une quantité mesurable d'unités $R^2{}_2SiO_{2/2}$, ou $R^3SiO_{3/2}$, et que la quantité totale de polyorganosiloxane ajoutée à la réaction entre A) et B) n'aboutisse pas à plus de 50% en moles d'unités D ou T dans le mélange réactionnel.
Le polyorganosiloxane peut comprendre des combinaisons d'unités M, D, T et Q, pourvu qu'au moins les unités D ou T soient présentes. Ainsi, le polyorganosiloxane peut être choisi parmi les silicones fluides, gommes, ou résines connues de l'art antérieur et comprenant des unités D ou T, ou leurs mélanges. Les unités D comprennent typiquement des groupes méthyle ou phényle ou leurs mélanges comme groupes $R^2$. Les unités T comprennent typiquement des groupes

méthyle ou phényle ou leurs mélanges comme groupes $R^3$. Le polyorganosiloxane peut être un polydiorganosiloxane fluide linéaire ayant une viscosité comprise entre 10 et 1000 cS ($mm^2/s$). Le polydiorganosiloxane fluide peut être une polydiméthylsiloxane, ou une polyméthylphénylsiloxane. Le polyorganosiloxane peut également être une résine organosilsesquioxane. La résine organosilsesquioxane est typiquement une résine méthylsilsesquioxane ou une résine phénylsilsesquioxane.

Les composants A), B) et optionnellement C) peuvent réagir par toute méthode connue de l'art antérieur pour agir sur les unités M, D, T et Q. De préférence cependant, les composants A), B) et optionnellement C) réagissent par une réaction de condensation en présence de catalyseur. La résine MQ est typiquement présente dans un solvant hydrocarboné aromatique ou siloxane. Des catalyseurs de réaction de condensation utilisables sont notamment des hydroxydes métalliques comme l'hydroxyde de potassium ou l'hydroxyde de sodium ; les sels métalliques comme les silanolates, les carboxylates et les carbonates ; les aminés ; les titanates comme le tétrabutyl titanate ; et leurs mélanges. Typiquement la réaction entre les composants A), B) et optionnellement C) est effectuée en chauffant le mélange réactionnel à des températures allant de 50 à 140°C, de préférence allant de 100 à 140°C. La réaction peut se dérouler en processus semi-continu, continu ou dans un batch.

De préférence, ladite résine de siloxane est obtenue par un procédé comprenant la réaction entre :

A) une résine MQ comprenant au moins 80 % en moles d'unités $(R^1{}_3SiO_{1/2})_a$ et $(SiO_{4/2})_d$

$R^1$ représentant un groupement méthyle,
a et d étant supérieurs à zéro,
le rapport a/d étant compris entre 0,5 et 1,5 ;

et
B) une résine de T propyle comprenant au moins 80 % en moles d'unités $(R^3SiO_{3/2})c$,

$R^3$ représentant un groupement propyle,
c étant supérieur à zéro,

où le ratio massique A/B est compris entre 95:5 et 15:85, de préférence le ratio massique A/B est de 30 :70.

Le rapport massique A/B dans la réaction est compris entre 95:5 et 15:85, de préférence entre 95 :5 et 20 :80, de préférence entre 90 :10 et 20 :80.

De préférence, le rapport massique A/B est égal à 85:15, ou 50:50, ou 30:70, ou 95:5. De préférence, le rapport massique A/B est égal à 30:70.

La quantité de composant C) peut varier, mais à la condition qu'elle aboutisse à une teneur inférieure à 30% en moles d'unités additionnelles D ou T, par rapport à la quantité molaire totale d'unités siloxane du mélange réactionnel.

La composition selon l'invention comprend une quantité de résine de siloxane, en poids de matière active (matière sèche), allant de 0,5 à 60% en poids, par rapport au poids total de la composition, de préférence de 3 à 60 % en poids, et mieux de 4 à 60% en poids par rapport au poids total de ladite composition.

Selon un mode particulier, la quantité de résine de siloxane, en poids de matière active (matière sèche) ira avantageusement de 3 à 60 % en poids, et mieux de 6 à 60% en poids par rapport au poids total de ladite composition. Ces teneurs sont notamment adaptées aux compositions sous forme anhydre et en particulier aux compositions sous forme de stick, telles que les rouges à lèvres.

Selon un autre mode particulier, la quantité de résine de siloxane, en poids de matière active (matière sèche) ira avantageusement de 3 à 30 % en poids, et mieux de 4 à 20% en poids par rapport au poids total de ladite composition. Ces teneurs sont notamment adaptées aux compositions sous forme d'émulsions, et en particulier aux compositions sous forme d'émulsions E/H, telles que les fonds de teint liquides.

## CHARGE

La composition selon l'invention comprend au moins une charge minérale.

Par charges minérales, il faut comprendre des particules incolores ou blanches, minérales, lamellaires ou non lamellaires. Les charges minérales peuvent être de toute forme, telle que notamment patatoïde, lamellaire, plaquettaire, sphérique ou oblongue. Elles peuvent être de toute forme cristallographique (par exemple feuillet, cubique, hexagonale, orthorhombique, La charge minérale est choisie parmi le talc, le mica, la silice, le kaolin, le nitrure de bore, le carbonate de calcium précipité, le carbonate et l'hydro-carbonate de magnésium, l'hydroxyapatite, les microsphères de silice creuses (Silica Beads® de Maprecos), ou les microcapsules de verre ou de céramique, l'argile, le quartz, la poudre de diamant naturel ou leur mélange.

Comme poudre de silice, on peut citer :

- les microsphères de silice poreuses vendues sous la dénomination SILICA BEADS SB-700 par la société MYOSHI ; "SUNSPHERE® H51", "SUNSPHERE® H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.

De préférence, la charge minérale est la silice, le talc ou leur mélange.
Parmi les charges sphériques, on préfère les silices, comme les microsphères de silice creuses, en particulier les SB700® de Miyoshi Kasei.
Selon une variante de l'invention, la composition comprend au moins une charge minérale de forme patatoïde (c'est-à-dire, une forme de surface présentant un contour irrégulier qui évoque la section longitudinale d'une pomme de terre), lamellaire ou leur mélange.
Les charges minérales peuvent être présentes dans la composition en une teneur pouvant varier dans une large mesure en fonction de la nature des charges minérales et également en fonction du produit désiré et/ou des effets désirés, cette quantité est à adapter par l'homme du métier.
La ou les charge(s) minérale(s) peu(ven)t être présente(s) dans la composition en une teneur allant de 0,01 à 50% en poids, notamment de 0,01 % à 30 % en poids, par rapport au poids total de la composition, de préférence allant de 0,01 % à 20 % en poids.
Plus spécifiquement, la ou les charge(s) minérale(s) peu(ven)t être présente(s) dans la composition en une teneur allant de 1 à 50% en poids, par rapport au poids total de la composition, et mieux de 2 à 35 % en poids.
Selon un mode préféré, la composition selon l'invention comprend en outre au moins une autre charge. Ladite au moins une autre charge peut être minérale ou organique. Il peut ainsi s'agir d'un mélange de charges minérales et organiques.
Selon une alternative, la composition selon l'invention peut contenir une charge minérale et une autre charge minérale, lesdites charges minérales étant telles que définies ci-dessus, et éventuellement au moins une charge organique, telle que définie ci-dessous.
Selon une autre alternative, la composition selon l'invention peut contenir une charge minérale et une charge organique.
Par charges organiques, il faut comprendre des particules incolores ou blanches, organiques, lamellaires ou non lamellaires. On peut notamment citer les poudres de polyamide (Nylon® ou Orgasol® de chez Arkema), les poudres de polymères acryliques, notamment les poudres de polyméthyl méthacrylate, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, les poudres de cellulose, de poly-β-alanine et de polyéthylène, les poudres de polymères de tétrafluoroéthylène (Téflon®), la lauroyl-lysine, l'amidon, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, notamment obtenues par polymérisation d'organopolysiloxane ayant au moins deux atomes d'hydrogène liés chacun à un atome de silicium et d'un organopolysiloxane comprenant au moins deux groupes à insaturation éthylénique (notamment deux groupes vinyles) en présence de catalyseur platine, ou encore les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Comme poudre de polymères acryliques, on peut citer :

- les poudres de polyméthacrylate de méthyle vendues sous la dénomination COVABEAD® LH85 par la société WACKHERR ;
- les poudres de poly méthacrylate de méthyle/diméthacrylate d'éthylène glycol vendues sous la dénomination DOW CORNING 5640 M1CROSPONGE® SKIN OIL ADSORBER par la société DOW CORNING ; GANZPEARL® GMP-0820 par la société GANZ CHEMICAL ;
- les poudres de polyméthacrylate d'allyle/diméthacrylate d'éthylène glycol vendues sous la dénomination POLY-PORE® L200, POLY-PORE® E200 par la société AMCOL ;
- les poudres de copolymère diméthacrylate d'éthylène glycol/méthacrylate de lauryle vendues sous la dénomination POLYTRAP® 6603 de la société DOW CORNING.

Comme poudre de silicone élastomère, on peut citer les poudres vendues sous les dénominations "Trefil® Powder E-505C", "Trefil® Powder E-506C" par la société DOW CORNING.
De préférence, la charge organique correspond aux poudres de polyamide.
La quantité de charges peut varier dans une large mesure en fonction de la nature de la charge et également en fonction du produit désiré et/ou des effets désirés, cette quantité est à adapter par l'homme du métier.

La ou les charge(s) peu(ven)t être présente(s) dans la composition en une teneur allant de 0,01 à 50% en poids, notamment de 0,01 % à 30 % en poids, par rapport au poids total de la composition, de préférence allant de 0,01 % à 20 % en poids.
Plus spécifiquement, la ou les charge(s) peu(ven)t être présente(s) dans la composition en une teneur allant de 1 à 50% en poids, par rapport au poids total de la composition, et mieux de 2 à 35 % en poids.
Dans le cas où la composition selon l'invention comprend des charges minérales et d'autres charges de type organiques, elles seront dans ladite composition dans un rapport pondéral charges minérales/charges organiques de préférence supérieur ou égal à 1.
La présente invention a également pour objet une composition, susceptible d'être mise en oeuvre dans un procédé selon l'invention comprenant, dans un milieu physiologiquement acceptable, au moins une résine de siloxane telle que définie ci-dessus et au moins une charge minérale telle que définie ci-dessus, comprenant éventuellement en outre au moins une charge organique telle que définie ci-dessus.
La composition selon l'invention peut comprendre un ou plusieurs autres composants, et en particulier les huiles, les composés pâteux, les cires, dures ou molles, les additifs rhéologiques, les matières colorantes, notamment des pigments ou des nacres traités ou non en surface par un agent hydrophobe, les polymères, notamment ceux à groupements saccharides ou carboxylates, ou leur mélange.

Polymère :

Les compositions selon l'invention peuvent contenir en outre au moins un polymère, filmogène ou non.
Dans la présente invention, on entend par « polymère filmogène », un polymère apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconée.
La composition peut comporter une phase aqueuse et le polymère additionnel peut être présent dans cette phase aqueuse. Dans ce cas celui-ci sera de préférence un polymère en dispersion ou un polymère amphiphile ou associatif.
Par «*polymère en dispersion*» on entend des polymères non solubles dans l'eau présents sous forme de particules de taille variable. Le polymère peut être réticulé ou non. La taille de particules moyenne est typiquement comprise entre 25 et 500nm, de préférence entre 50 et 200 nm. Les polymères en dispersion aqueuse suivants peuvent être utilisés : Ultrasol 2075 de Ganz Chemical, Daitosol 5000AD de Daito Kasei, Avalure UR 450 de Noveon, DYNAMX de National Starch, Syntran 5760 de Interpolymer, Acusol OP 301 de Rohm&Haas, Neocryl A 1090 de Avecia.
Les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Soltex OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges, sont d'autres exemples de dispersion aqueuse de particules de polymères filmogènes hydrodispersibles.
Par «*polymères amphiphiles ou associatifs*» on entend des polymères comportant une au plusieurs partie hydrophiles qui les rendent partiellement solubles dans l'eau et une ou plusieurs parties hydrophobes par lesquelles les polymères s'associent ou interagissent. Les polymères associatifs suivants peuvent être utilisés : Nuvis FX1100 de Elementis, Aculyn 22, Aculyn 44, Aculyn 46 de Rohm&Haas, Viscophobe DB1000 de Amerchol. Les copolymères diblocs constitués d'un bloc hydrophile (polyacrylate, polyéthylène glycol) et d'un bloc hydrophobe (polystyrène, polysiloxane, peuvent également être utilisés.
Des polymères solubles dans une phase aqueuse contenant les particules monodisperses pourront être évités car ils peuvent provoquer une agrégation des particules monodisperses. Le polymère filmogène peut ainsi être non soluble dans une telle phase aqueuse.
La composition peut comporter une phase huileuse et le polymère filmogène peut être présent dans cette phase huileuse. Le polymère pourra alors être en dispersion ou en solution. Les polymères de type NAD (non aqueous dispersion) ou des microgel (par exemple les KSG) peuvent être utilisés, ainsi que les polymères du type polyamide siliconé ou les copolymères à base de styrène (Kraton, Regalite).

Comme exemples de dispersions non aqueuses de polymère filmogène lipodispersibles sous forme de dispersions non aqueuses de particules de polymère dans une ou plusieurs huiles de silicone et/ou hydrocarbonées et pouvant être stabilisées en leur surface par au moins un agent stabilisant, notamment un polymère séquencé, greffé ou statistique, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser.

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C30, de préférence en C1-C20, des (méth)acrylates d'aryle, en particulier d'aryle en C6-C10, des (méth)acrylates d'hydroxyalkyle, en particulier d'hydroxyalkyle en C2-C6.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.
Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide

isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO3M, avec M représentant un atome d'hydrogène, un ion ammonium NH4+ ou un ion métallique, comme par exemple un ion Na+, Li+, K+, Mg2+, Ca2+, Cu2+, Fe2+, Fe3+. On peut utiliser notamment un monomère aromatique bifonctionnel comportant un tel groupement -SO3M.

Le noyau aromatique du monomère aromatique bifonctionnel portant en outre un groupement -SO3M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO3M: l'acide sulfoisophtalique, l'acide sulfotéréphtalique, l'acide sulfophtalique, l'acide 4-sulfonaphtalène-2,7-dicarboxylique.

A titre d'exemple, le polymère filmogène peut être un polymère solubilisé dans une phase grasse liquide comprenant des huiles ou solvants organiques (on dit alors que le polymère filmogène est un polymère liposoluble). De préférence, la phase grasse liquide comprend une huile volatile, éventuellement en mélange avec une huile non volatile.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme exemple de polymères filmogènes liposolubles, on peut citer les copolymères d'ester vinylique et au moins un autre monomère qui peut être un ester vinylique, notamment le néodécanoate de vinyle, le benzoate de vinyle et le t-butyl benzoate de vinyle, une $\alpha$-oléfine, un alkylvinyléther, ou un ester allylique ou méthallylique.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisées par la société Wacker sous la référence Resin MK tels que la Belsil PMS MK, ou par par la société SHIN-ETSU sous les références KR-220L. A titre d'exemples de résines polypropylsilsesquioxanes commercialement disponibles, on peut citer celles qui sont commercialisés sous la référence DC670 par la société Dow Corning.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination KF-7312J par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

A titre d'exemple, le polymère filmogène peut être un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

Le polymère filmogène peut être choisi parmi les polymères et/ou copolymères blocs ou statiques comportant notamment les polyuréthanes, polyacryliques, les silicones, les polymères fluorés, les gommes butyliques, les copolymères d'éthylènes, gommes naturelles et les alcools polyvinyliques et leurs mélanges. Les monomères des copolymères blocs ou statiques comprenant au moins une association de monomères dont le polymère résulte à une température de transition vitreuse inférieure à la température ambiante (25 °C) peuvent être choisis parmi notamment le butadiène, l'éthylène, le propylène, l'acrylique, le méthacrylique, l'isoprène, l'isobutène, une silicone et leurs mélanges.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

A titre d'autres exemples de système filmogène utilisable dans les compositions selon l'invention, on peut citer les systèmes dans lequel le film se forme in-situ au moment de l'application de la composition ou d'un mélange de compositions contenant deux composés siliconés réagissant lorsqu'ils sont mis en contact l'un de l'autre. De tels systèmes sont décrits notamment dans la demande WO 2007/071706. Des systèmes de ce type sont également décrits dans les demandes US2007/142575 ou US 2007/142599.

**<u>Autres polymères :</u>**

Les compositions selon l'invention peuvent contenir un élastomère, notamment un élastomère de silicone polyglycérolé. A titre d'exemple, on utilise un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

Comme élastomères de silicone polyglycérolés, on peut utiliser ceux vendus sous les dénominations "KSG-710", "KSG-810", "KSG-820", "KSG-830", "KSG-840" par la société Shin Etsu.

Les compositions selon l'invention peuvent en outre comprendre un élastomère de silicone émulsionnant additionnel.

A titre d'exemples, on utilise des élastomères polyoxyalkylénés tels que décrits notamment décrits dans les brevets US5236986, US5412004, US5837793, US5811487.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.

Ces élastomères particuliers, lorsqu'ils sont en association avec les résines selon l'invention, peuvent permettre d'améliorer les propriétés de non transfert et de confort (souplesse) des compositions les comprenant.

Les compositions selon l'invention peuvent comprendre en outre un élastomère non émulsionnant.

Des élastomères non-émulsionnants sont notamment décrits dans les demandes JP-A-61-194009, EP-A-242219, EP-A-285886, EP-A-765656.

Comme élastomères non-émulsionnants sphériques, on peut utiliser ceux vendus sous les dénominations "DC 9040", "DC9041 ", "DC 9509", "DC9505", "DC 9506" par la société Dow Corning.

L'élastomère de silicone non émulsionnant sphérique peut se présenter également sous forme de poudre d'organopolysiloxane réticulé élastomère enrobée de résine de silicone, notamment de résine silsesquioxane, comme décrit par exemple dans le brevet US5538793. De tels élastomères sont vendus sous les dénomination "KSP-100", "KSP-101", "KSP-102", "KSP-103", KSP-104", "KSP-105" par la société Shin Etsu.

D'autres organopolysiloxanes réticulés élastomère sous forme de poudres sphériques peuvent être des poudres de silicone hybride fonctionnalisé par des groupes fluoroalkyle, notamment vendues sous la dénomination "KSP-200" par la société Shin Etsu ; des poudres de silicones hybride fonctionnalisé par des groupes phényl, notamment vendues sous la dénomination "KSP-300" par la société Shin Etsu.

On peut également utiliser dans les compositions selon l'invention des élastomères de silicones avec groupement MQ, tels que ceux vendus par la Société Wacker sous les dénominations Belsil RG100, Belsil RPG33 et préférentiellement RG80. Ces élastomères particuliers, lorsqu'ils sont en association avec les résines selon l'invention, peuvent permettre d'améliorer les propriétés de non transfert des compositions les comprenant.

**<u>Agents structurants :</u>**

La composition selon l'invention peut comprendre au moins un agent structurant.

On entend par agent structurant un composé apte à augmenter la viscosité de la composition. L'agent structurant permet notamment d'obtenir une composition pouvant présenter une texture allant des textures fluides à solides.

L'agent structurant peut être présent dans la composition en une teneur allant de 0,05 % à 40 % en poids, par rapport au poids total de la composition, de préférence allant de 0,1 % à 30 % en poids, et préférentiellement allant de 0,1 % à 25 % en poids.

L'agent structurant peut être notamment choisi parmi les épaississants (épaississants de milieux huileux ; épaississants de milieu aqueux), les organogélateurs, les cires, les composés pâteux, les gommes.

L'agent épaississant de milieu aqueux peut être choisi parmi :

- les argiles hydrophiles,
- la silice pyrogénée hydrophile.
- les épaississants cellulosiques hydrosolubles
- les gommes de guar, de xanthane, de caroube, de scléroglucane, de gellane, de rhamsan, de karaya, de carraghénane
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

De tels agents épaississants sont notamment décrits dans les demandes EP-A-1400234.

L'agent épaississant de milieu huileux peut être choisi parmi

- Les silicones carboxylates
- Les silicones sacharrides
- les argiles organophiles,
- les silices pyrogénées hydrophobes
- les gommes de guar alkylées (avec groupe alkyle en C1-C6), telles que celles décrites dans EP-A-708114 ;

- les celluloses hydrophobes
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619; en particulier les résines de polyamides (notamment comprenant des groupes alkyles ayant de 12 à 22 atomes de carbone) telles que celles décrites dans US-A-5783657.
- les résines de polyamides siliconées telles que décrites dans la demande EP-A-1266647 , dans la demande de brevet français déposée sous le n° 0216039.

De tels agents épaississants sont notamment décrits dans les demandes EP-A-1400234.

Les organogélateurs peuvent être choisis parmi ceux décrits dans la demande WO-A-03/105788. A titre d'exemples, on peut citer notamment :

- Les dérivés de bis-urées de formule générale (II) :

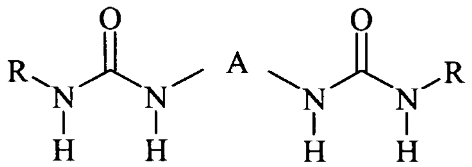

(II)

dans laquelle :

- A est un groupement de formule :

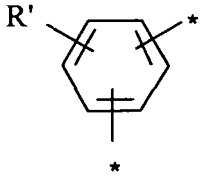

avec R' étant un radical alkyle $C_1$ à $C_4$ linéaire ou ramifié et les * symbolisant les points de rattachement du groupement A à chacun des deux atomes d'azote du reste du composé de formule générale (1), et
- R est un radical alkyle en $C_6$ à $C_{15}$, mono-ramifié, non cyclique, saturé ou insaturé et dont la chaîne hydrocarbonée est éventuellement interrompue par 1 à 3 hétéroatomes choisis parmi O, S et N, ou

l'un de ses sels ou isomères notamment décrits dans la demande de brevet FR-A-2892303

- Les dérivés de bis-urées siliconés de formule générale (III) ou l'un de ses sels et/ou isomères :

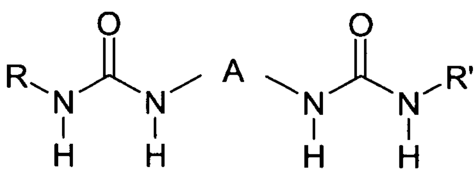

(III)

dans laquelle :

- A est un groupement de formule (IV) :

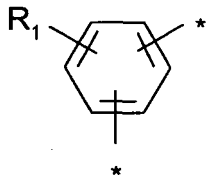

avec R1 étant un radical alkyle $C_1$ à $C_4$ linéaire ou ramifié, et les * symbolisant les points de rattachement du groupement A à chacun des deux atomes d'azote du reste du composé de formule générale (I), et

- R et R', identiques ou différents, sont choisis parmi :

  - i) les radicaux de formule (V) :

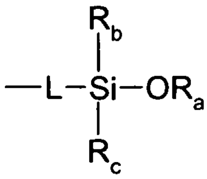

(V)

dans laquelle :

- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
- Ra est :

  a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
  b) un radical siliconé de formule :

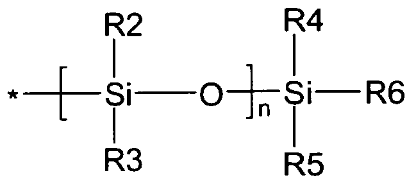

  avec n étant compris entre 0 et 100, notamment entre 1 et 80, voire 2 à 20;
  et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;

- Rb et Rc sont, indépendamment l'un de l'autre, choisis parmi:

  a) les radicaux carbonés, notamment hydrocarbonés (alkyles), linéaires, ramifiés et/ou cycliques, saturés ou insaturés, comprenant 1 à 18 atomes de carbone, et pouvant comprendre 1 à 4 hétéroatomes choisis parmi N, O, Si et S;
  b) les radicaux de formule :

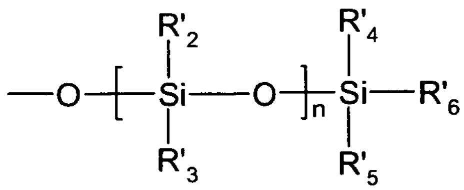

avec n étant compris entre 0 et 100, notamment entre 1 et 80, voire 2 à 20;
et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
et

- ii) les radicaux alkyle en $C_1$ à $C_{30}$, linéaire, ramifié et/ou cyclique, saturé ou insaturé, et comprenant éventuellement 1 à 3 hétéroatomes choisis parmi O, S, F et N;
  étant entendu que l'un au moins des radicaux R et/ou R' est de formule (III) tels que ceux décrits dans la demande de brevet FR-A-2900819.

- Les dérivés de bis-urées décrits dans la demande de brevet FR-A-28994476.

Les agents structurants peuvent être constitués de cires. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.
En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.
Les cires peuvent être hydrocarbonées, fluorées et/ou siliconées et être d'origine végétale, minérale, animale et/ou synthétique. En particulier, les cires présentent une température de fusion supérieure à 30 °C et mieux supérieure à 45 °C.
Comme cire utilisable dans la composition de l'invention, on cite de préférence la cire d'abeilles, la cire de Carnauba ou de Candellila, la paraffine, les cires microcristallines, la cire de son de riz, les cires d'Olive (photowax olive 14L48, photowax olive 18L57) la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. A titre indicatif, la composition peut contenir de 0,1 à 50 % en poids de cires, par rapport au poids total de la composition et mieux de 1 à 30 % en poids.
Les gommes sont généralement des polydiméthylsiloxanes (PDMS) à haut poids moléculaire ou des gommes de cellulose ou des polysaccharides.

Tensioactifs

La composition selon l'invention peut comprendre au moins un tensioactif.
Le tensioactif peut être lipophile et/ou hydrophile, utilisé seul ou en couplage Le tensioactif peut être choisi parmi les tensioactifs non ioniques, anioniques, cationiques, amphotères.
Le tensioactif non ionique peut être choisi parmi :

- un alkyl C8-C22 diméthicone copolyol , c'est-à-dire un poly méthyl alkyl(C8-C22) diméthyl méthyl siloxane oxypropyléné et/ou oxyéthyléné.

L'alkyl C8-C22 diméthicone copolyol est avantageusement un composé de formule (VI) suivante :

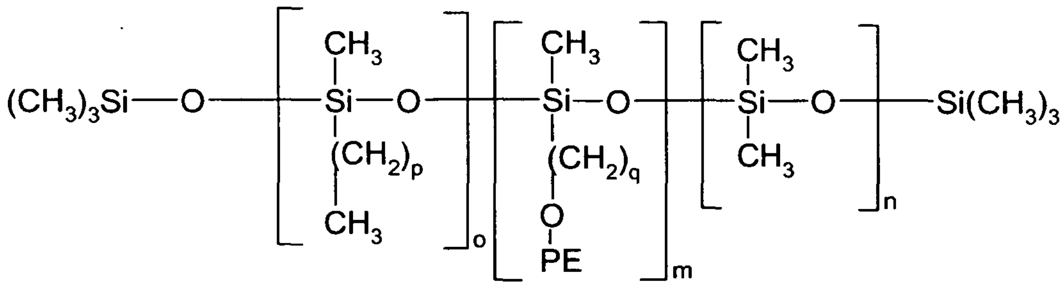

(VI)

dans laquelle :

- PE représente (-C2H4O)x-(C3H6O)y-R, R étant choisi parmi un atome d'hydrogène et un radical alkyle de 1 à 4 atomes de carbone, x allant de 0 à 100 et y allant de 0 à 80, x et y n'étant pas simultanément 0
- m allant de 1 à 40
- n allant de 10 à 200
- o allant de 1 à 100
- p allant de 7 et 21
- q allant de 0 à 4

et de préférence :

R=H
m = 1 à 10
n = 10 à 100
o = 1 à 30
p= 15
q = 3

Comme alkyl C8-C22 diméthicone copolyol, on peut citer le cétyl diméthicone copolyol comme le produit commercialisé sous la dénomination Abil EM-90 par la société Goldschmidt.

- un diméthicone copolyol, c'est-à-dire un polydiméthyl méthyl siloxane oxypropylèné et/ou oxyéthylèné. Il ne contient pas de groupement alkyle à longue chaîne de plus de 8 atomes de carbone, notamment en C8-C22.

On peut utiliser comme diméthicone copolyol ceux répondant à la formule (VII) suivante :

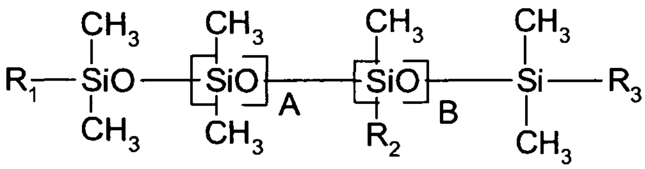

(VII)

dans laquelle :

R1, R2, R3, indépendamment les uns des autres, représentent un radical alkyle en C1-C6 ou un radical -(CH2)x - (OCH2CH2)y - (OCH2CH2CH2)z - OR4, au moins un radical R1, R2 ou R3 n'étant pas un radical alkyle ; R4 étant un hydrogène, un radical alkyle en C1-C3 ou un radical acyle en C2-C4 ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (VII), R1 = R3 = radical

méthyle, x est un nombre entier allant de 2 à 6 et y est un nombre entier allant de 4 à 30. R4 est en particulier un hydrogène.

On peut citer, à titre d'exemple composés de formule (VII), les composés de formule (VIII) :

$$\begin{array}{l} (CH3)3SiO - [(CH3)2SiO]A - (CH3SiO)B - Si(CH3)3 \\ \qquad\qquad\qquad\qquad\qquad\qquad\qquad\quad | \\ \qquad\qquad\qquad\qquad\qquad\qquad\qquad (CH2)2\text{-}(OCH2CH2)y\text{-}OH \end{array} \qquad (VIII)$$

dans laquelle A est un nombre entier allant de 20 à 105, B est un nombre entier allant de 2 à 10 et y est un nombre entier allant de 10 à 20.

On peut également citer à titre d'exemple de composés siliconés de formule (VII), les composés de formule (IX) :

HO - (CH2CH2O)y-(CH2)3 - [(CH3)2SiO]A' - [(CH3)2Si] - (CH2)3 - (OCH2CH2)y - OH

dans laquelle A' et y sont des nombres entiers allant de 10 à 20.

On peut utiliser comme diméthicone copolyol ceux vendus sous les dénominations DC 5329, DC 7439-146, DC 2-5695, Q4-3667 par la société Dow Coming ; KF-6013, KF-6015, KF-6016, KF-6017 par la société Shin-Etsu.
Les composés DC 5329, DC 7439-146, DC 2-5695 sont des composés de formule (VIII) où respectivement A est 22, B est 2 et y est 12 ; A est 103, B est 10 et y est 12 ; A est 27, B est 3 et y est 12.

Comme tensioactif non ionique, on peut également citer les esters d'acides gras de polyols comme les mono-, di-, tri- ou sesqui-oléates ou stéarates de sorbitol ou de glycérol, les laurates de glycérol ou de polyéthylène glycol ; les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés.

Comme tensioactif anionique, on peut citer les carboxylates (2-(2-Hydroxyalkyloxy) acétate de sodium), les dérivés des aminoacides (N-acylglutamates, N-acylglycinates, acylsarcosinates), les alkyl sulfates, les alkyl éther sulfates et leurs dérivés oxyéthylénés, les sulfonates, les iséthionates et N-acyliséthionates, les taurates et N-acyl N-méthyltaurates, les sulfosuccinates, les alkylsulfoacétates, les phosphates et alkylphosphates, les polypeptides, les dérivés anioniques d'alkyl polyglycoside (acyl-D-galactoside uronate), les savons d'acides gras, et leurs mélanges.

Comme tensioactif amphotère et zwitterionique, on peut utiliser les bétaïnes, les N-alkylamidobétaïnes et leurs dérivés, les dérivés de la glycine, les sultaïnes, les alkyl polyaminocarboxylates, les alkylamphoacétates, et leurs mélanges.

De tels tensioactifs sont notamment décrits dans la demande WO-A-02/056854.

Le tensioactif peut être présent dans la composition selon l'invention en une teneur allant de 0,1 % à 10 % en poids, par rapport au poids total de la composition, et de préférence allant de 0,5 % à 8 % en poids, et préférentiellement allant de 0,5 % à 7 % en poids.

<u>Matières colorantes :</u>

La composition selon l'invention peut comprendre en outre au moins une matière colorante.
La matière colorante peut être choisie parmi les matières colorantes pulvérulentes (notamment les pigments et les nacres), les matières colorantes hydrosolubles ou liposolubles.

En particulier, les pigments peuvent être blancs ou colorés, minéraux et/ou organiques. On peut citer, parmi les pigments minéraux, le dioxyde de titane, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

Les colorants liposolubles, synthétiques ou naturels sont, par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les particules solides, telles que les matières colorantes pulvérulentes (pigments et nacres), peuvent être traitées en surface totalement ou partiellement avec un composé de nature siliconée, un composé de nature fluorée, un composé de nature fluoro-siliconée, un acide gras ou acide aminé ou un de leurs mélanges.

Selon un mode de réalisation, les compositions, en particulier les compositions de maquillage ou de soin de la peau et notamment les fonds de teint, peuvent comprendre au moins une particule solide (plus spécifiquement pigments et/ou nacres) traitée en surface totalement ou partiellement avec un composé de nature fluorée, notamment pour améliorer la tenue de la couleur et de la matité.

L'agent de traitement hydrophobe peut être choisi parmi les silicones comme les méthicones, les diméthicones, les perfluoroalkylsilanes, les perfluoroalkyl silazanes, le triéthoxy caprylylsilane, le triéthoxysilyléthyl polydiméthylsiloxyéthyl hexyl diméthicone ; les acides gras comme l'acide stéarique ; les savons métalliques comme le dimyristate d'aluminium, le sel d'aluminium du glutamate de suif hydrogéné ; les perfluoroalkyl phosphates, les polyoxydes d'hexafluoropropylène, les polyorganosiloxanes comprenant des groupes perfluoroalkylles perfluoropolyéthers, les polymères acryliques greffés silicone (notamment décrits dans la demande JP-A-05-339125) ; les acides aminés ; les acides aminés N-acylés ou leurs sels ; la lécithine, le trisostéaryle titanate d'isopropyle, le sébaçate d'isostéaryle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine.

Les agents de surface fluorés peuvent être choisis parmi les phosphates de perfluoroalkyle, les perfluoropolyéthers, les polytétrafluopolyéthylène (PTFE) et les perfluoroalcanes.

Les perfluoropolyéthers sont notamment décrits dans la demande de brevet EP-A-486135, et vendus sous les dénominations commerciales FOMBLIN par la société MONTEFLUOS.

Des phosphates de perfluoroalkyle sont en particulier décrits dans la demande JP H05-86984. Les phosphate-diéthanol amine de perfluoroalkyle commercialisés par Asahi Glass sous la référence AsahiGuard AG530 peuvent être utilisés.

Parmi les perfluoroalcanes linéaires, on peut citer les perfluorocycloalcanes, les perfluoro(alkylcycloalcanes), les perfluoropolycycloalcanes, les hydrocarbures perfluorés aromatiques (les perfluoroarènes) et les composés organo perfluorés hydrocarbonés comportant au moins un hétéroatome.

Parmi les perfluoroalcanes, on peut citer la série des alcanes linéaires tels que le perfluorooctane, le perfluorononane ou le perfluorodécane.

Parmi les perfluorocycloalcanes et les perfluoro(alkylcycloalcanes), on peut citer la perfluorodécaline vendue sous la dénomination de "FLUTEC PP5 GMP" par la Société RHODIA, la perfluoro(méthyldécaline), les perfluoro(C3-C5 alkylcyclohexanes) tels que le perfluoro(butylcyclohexane).

Parmi les perfluoropolycycloalcanes on peut citer les dérivés de bicyclo [3.3.1] nonane tel que le perfluorotriméthylbicyclo [3.3.1] nonane, les dérivés de l'adamantane tels que le perfluorodiméthyladamantane et les dérivés perfluorés de phénanthrène hydrogéné tel que le tétracosafluoro-tétradécahydrophénanthrène.

Parmi les perfluoroarènes, on peut citer les dérivés perfluorés du naphtalène comme le perfluoronaphtalène et le perfluorométhyl-1-napthtalène.

A titre d'exemple de références commerciales de pigments traités avec un composé fluoré, on peut citer :

- L'oxyde de fer jaune/phosphate de perfluoroalkyle vendu sous la référence PF 5 Yellow 601 par la société Daito Kasei,
- L'oxyde de fer rouge/phosphate de perfluoroalkyle vendu sous la référence PF 5 Red R 516L par la société Daito Kasei,
- L'oxyde de fer noir/phosphate de perfluoroalkyle vendu sous la référence PF 5 Black BL 100 par la société Daito Kasei,
- Le dioxyde de titane/phosphate de perfluoroalkyle vendu sous la référence PF 5 TiO2 CR 50 par la société Daito Kasei,
- L'oxyde de fer jaune/perfluoropolymethylisopropylether vendu sous la référence Iron oxyde yellow BF-25-3 par la société Toshiki,
- Le DC Red 7/perfluoropolymethylisopropylether vendu sous la référence D&C Red 7 FHC par la société Cardre Inc.,
- Le DC Red 6/PTFE vendu sous la référence T 9506 par la société Warner-Jenkinson,

Les matières colorantes, en particulier les pigments peuvent être présents dans la composition en une teneur allant de 0,1 % à 30 % en poids, par rapport au poids total de la composition, de préférence allant de 0,5 % à 20 % en

poids, et préférentiellement allant de 1 % à 10 % en poids.

**Fibres :**

La composition peut comprendre en outre des fibres.

Par "fibre", il faut comprendre un objet de longueur L et de diamètre D tel que L soit supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm et mieux de 0,3 mm à 3 mm. Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. De préférence, les fibres selon l'invention ont un titre choisi dans la gamme allant de 0,01 à 10 à deniers, de préférence de 0,1 à 2 deniers et mieux de 0,3 à 0,7 deniers.

De telles fibres sont notamment décrites dans la demande de brevet français déposée sous le n° 0450074, les demandes FR-A-2844710, EP-A-1201221.

Les fibres peuvent être présentes dans la composition en une teneur allant de 0,1 % à 30 % en poids, par rapport au poids total de la composition, de préférence allant de 0,1 % à 20 % en poids, et préférentiellement allant de 0,1 % à 10 % en poids.

Par « milieu physiologiquement acceptable », on entend désigner un milieu convenant particulièrement à l'application d'une composition de l'invention sur la peau ou ses phanères. Le milieu physiologiquement acceptable est généralement adapté à la nature du support sur lequel doit être appliquée la composition, ainsi qu'à l'aspect sous lequel la composition doit être conditionnée.

La composition selon l'invention peut se présenter sous diverses formes, notamment sous forme de poudres (libres ou compactes), de composition anhydre, de dispersion, émulsion, telle que notamment eau/huile ou eau/cire, huile/eau, multiples ou cire/eau, ou encore sous forme de gel.

Une composition de l'invention est de préférence une émulsion, en particulier directe ou inverse, ou une composition anhydre.

Une dispersion peut être effectuée en phase aqueuse ou en phase huileuse.

Une émulsion peut posséder une phase continue huileuse ou aqueuse. Une telle émulsion peut être, par exemple, une émulsion inverse (E/H) ou directe (H/E), ou encore une émulsion multiple (E/H/E ou H/E/H).

Dans le cas des émulsions, les émulsions inverses (E/H) sont préférentielles.

Une composition anhydre est une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.

La composition selon l'invention peut se présenter sous la forme d'un fluide par exemple pâteux ou liquide. Elle peut également se présenter sous forme de poudre libre ou compacte, de pâte souple, d'une crème. Par exemple, elle peut être une émulsion huile-dans-eau, eau-dans-huile ou multiple, une émulsion solide notamment de type eau dans huile, un gel notamment anhydre, solide ou souple, sous forme de poudre libre ou compactée et même sous forme biphasique.

La composition considérée selon l'invention se présente généralement sous la forme d'une composition de maquillage et/ou de soin des matières kératiniques, par exemple d'un fond de teint notamment à appliquer sur le visage ou le cou, d'un produit anti-cernes, d'un correcteur de teint, d'une crème teintée, d'un fard à joues, d'une composition de maquillage pour le corps.

**Phase aqueuse**

La composition selon l'invention peut comprendre au moins une phase aqueuse.

La phase aqueuse comprend de l'eau. Une eau convenant à l'invention peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante -25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol,

l'hexylène glycol, le dipropylène glycol, le diéthylène glycol ; les éthers de glycol (ayant notamment de 3 à 16 atomes de carbone) tels que les alkyl($C_1$-$C_4$)éther de mono, di- ou tripropylène glycol, les alkyl($C_1$-$C_4$)éthers de mono, di- ou triéthylène glycol, et leurs mélanges.
La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

En particulier, une composition de l'invention peut comprendre une phase aqueuse en une teneur variant de 1 % à 80 % en poids, notamment de 5 % à 50 %, et plus particulièrement de 10 % à 45 % en poids par rapport au poids total de la composition.

Selon un autre mode de réalisation, une composition de l'invention peut être anhydre.
Une composition anhydre peut comprendre moins de 5 % en poids d'eau, par rapport au poids total de la composition, et en particulier moins de 3 %, notamment moins de 2 %, et plus particulièrement moins de 1 % en poids d'eau par rapport au poids total de la composition. Plus particulièrement, une composition anhydre peut être dépourvue d'eau.

**Phase grasse**

Une composition cosmétique conforme à la présente invention peut comprendre au moins une phase grasse liquide et/ou solide.
En particulier, une composition de l'invention peut comprendre au moins une phase grasse liquide, notamment au moins une huile comme mentionnée ci-après.
On entend par huile, tout corps gras sous forme liquide à température ambiante (20-25 °C) et à pression atmosphérique.
Une composition de l'invention peut comprendre une phase grasse liquide en une teneur variant de 1 à 90 %, en particulier de 5 à 80 %, en particulier de 10 à 70 %, et plus particulièrement, de 20 à 50 % en poids par rapport au poids total de la composition.
La phase huileuse convenant à la préparation des compositions cosmétiques selon l'invention peut comprendre des huiles hydrocarbonées, siliconées, fluorées ou non, ou leurs mélanges. Les huiles peuvent être volatiles ou non volatiles. Elles peuvent être d'origine animale, végétale, minérale ou synthétique.
Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa ($10^{-3}$ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).
Au sens de la présente invention, on entend par « huile non volatile », une huile ayant une pression de vapeur inférieure à 0,13 Pa.
Au sens de la présente invention, on entend par « huile siliconée », une huile comprenant au moins un atome de silicium, et notamment au moins un groupe Si-O.
On entend par « huile fluorée », une huile comprenant au moins un atome de fluor.
On entend par « huile hydrocarbonée », une huile contenant principalement des atomes d'hydrogène et de carbone.
Les huiles peuvent éventuellement comprendre des atomes d'oxygène, d'azote, de soufre et/ou de phosphore, par exemple, sous la forme de radicaux hydroxyle ou acide.

Huiles volatiles

Les huiles volatiles peuvent être choisies parmi les huiles hydrocarbonées ayant de 8 à 16 atomes de carbone, et notamment les alcanes ramifiés en $C_8$-$C_{16}$ (appelées aussi isoparaffines), comme l'isododécane (encore appelé 2,2,4,4,6-pentaméthylheptane), l'isodécane, l'isohexadécane, et, par exemple, les huiles vendues sous les noms commerciaux d'ISOPARS® ou de PERMETHYLS®.
Comme huiles volatiles, on peut aussi utiliser les silicones volatiles, comme, par exemple, les huiles de silicones linéaires ou cycliques volatiles, notamment, celles ayant une viscosité ≤ 8 centistokes (cSt) ($8 \times 10^{-6}$ $m^2/s$), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées, telles que le nonafluorométhoxybutane ou le perfluorométhylcyclopentane, et leurs mélanges.
Selon un mode de réalisation, une composition de l'invention peut comprendre de 1 % à 80 % en poids, voire de 5 % à 70 % en poids, voire de 10 % à 60 % en poids, et notamment de 15 % à 50 % en poids d'huile volatile par rapport au poids total de la composition.

Huiles non volatiles

Les huiles non volatiles peuvent, notamment, être choisies parmi les huiles hydrocarbonées, fluorées et/ou les huiles siliconées non volatiles.
Comme huile hydrocarbonée non volatile, on peut notamment citer :

- les huiles hydrocarbonées d'origine animale, telle que le perhydrosqualène,
- les huiles hydrocarbonées d'origine végétale, telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de $C_4$ à $C_{36}$, et, notamment, de $C_{18}$ à $C_{36}$, ces huiles pouvant être linéaires ou ramifiées, saturées ou insaturées ; ces huiles peuvent, notamment, être des triglycérides héptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de canola, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol, l'huile de ricin, et l'huile de watermelon, et leurs mélanges, ou encore des triglycérides d'acides caprylique/caprique, comme ceux vendus par la société STEARINERIES DUBOIS ou ceux vendus sous les dénominations MIGLYOL 810®, 812® et 818® par la société DYNAMIT NOBEL,
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone ;
- les esters de synthèse, comme les huiles de formule $R_1COOR_2$, dans laquelle $R_1$ représente un reste d'un acide gras linéaire ou ramifié comportant de 1 à 40 atomes de carbone, et $R_2$ représente une chaîne hydrocarbonée, notamment, ramifiée contenant de 1 à 40 atomes de carbone à condition que $R_1 + R_2$ soit ≥ 10. Les esters peuvent être, notamment, choisis parmi les esters d'alcool et d'acide gras, comme par exemple : l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol, et leurs mélanges, les benzoates d'alcools en $C_{12}$-$C_{15}$, le laurate d'hexyle, les esters de l'acide néopentanoïque, comme le néopentanoate d'isodécyle, le néopentanoate d'isotridécyle, le néopentanoate d'isostéaryle, le néopentanoate d'octyldocécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;

- les esters de polyols et les esters du pentaérythritol, comme le tétrahydroxystéarate/tétraisostéarate de dipentaérythritol,
- les esters de dimères diols et de dimères diacides, tels que les Lusplan DD-DA5® et Lusplan DD-DA7®, commercialisés par la société NIPPON FINE CHEMICAL et décrits dans la demande US 2004-175338,
- les copolymères de dimère diol et de dimère diacide et leurs esters, tels que les copolymères dimères dilinoleyl diol/dimères dilinoléiques et leurs esters, comme par exemple le Plandool-G,
- les copolymères de polyols et de dimères diacides, et leurs esters, tels que le Hailuscent ISDA, ou le copolymère d'acide dilinoléique/butanediol,
- les alcools gras liquides à température ambiante à chaîne carbonée ramifiée et/ou insaturée ayant de 12 à 26

atomes de carbone, comme le 2-octyldodécanol, l'alcool isostéarylique, l'alcool oléique, le 2-hexyldécanol, le 2-butyloctanol, et le 2-undécylpentadécanol,

- les acides gras supérieurs en $C_{12}$-$C_{22}$, tels que l'acide oléique, l'acide linoléique, l'acide linolénique, et leurs mélanges, et
- les carbonates de di-alkyle, les 2 chaînes alkyles pouvant être identiques ou différentes, tels que le dicaprylyl carbonate commercialisé sous la dénomination CETIOL CC®, par COGNIS,
- les huiles de masse molaire élevée ayant, en particulier, une masse molaire allant d'environ 400 à environ 10 000 g/mol, en particulier, d'environ 650 à environ 10 000 g/mol, en particulier, d'environ 750 à environ 7500 g/mol, et plus particulièrement, variant d'environ 1000 à environ 5000 g/mol. Comme huile de masse molaire élevée utilisable dans la présente invention, on peut notamment citer les huiles choisies parmi :

    - les polymères lipophiles,
    - les esters d'acides gras linéaires ayant un nombre total de carbone allant de 35 à 70,
    - les esters hydroxylés,
    - les esters aromatiques,
    - les esters d'alcools gras ou d'acides gras ramifiés en $C_{24}$-$C_{28}$,
    - les huiles siliconées,
    - les huiles d'origine végétale,
    - et leurs mélanges.

- les huiles fluorées éventuellement partiellement hydrocarbonées et/ou siliconées comme les huiles fluorosiliconées, les polyéthers fluorés, les silicones fluorées telles que décrit dans le document EP-A-847 752;
- les huiles siliconées comme les polydiméthylsiloxanes (PDMS) non volatiles, linéaires ou cycliques ; les polydiméthylsiloxanes comportant des groupements alkyl, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et
- leurs mélanges.

Selon un mode de réalisation particulier, la phase grasse de la composition selon l'invention peut ne contenir que des composés volatils.

Une composition selon l'invention peut comprendre au moins un composé pâteux.

La présence d'un composé pâteux peut permettre de conférer avantageusement un confort amélioré lors du dépôt d'une composition de l'invention sur les fibres kératiniques.

Un tel composé peut être avantageusement choisi parmi la lanoline et ses dérivés ; les composés siliconés polymériques ou non ; les composés fluorés polymériques ou non ; les polymères vinyliques, notamment les homopolymères d'oléfines ; les copolymères d'oléfines ; les homopolymères et copolymères de diènes hydrogénés ; les oligomères linéaires ou ramifiés, homo ou copolymères de (méth)acrylates d'alkyles ayant de préférence un groupement alkyle en $C_8$-$C_{30}$ ; les oligomères homo et copolymères d'esters vinyliques ayant des groupements alkyle en $C_8$-$C_{30}$ ; les oligomères homo et copolymères de vinyléthers ayant des groupements alkyle en $C_8$-$C_{30}$ ; les polyéthers liposolubles résultant de la polyéthérification entre un ou plusieurs diols en $C_2$-$C_{10}$, en particulier, en $C_2$-$C_{50}$ ; les esters d'acide ou d'alcool gras ; et leurs mélanges.

Parmi les esters, on peut notamment citer :

- les esters d'un glycérol oligomère, notamment, les esters de diglycérol, comme le triisostéarate de polyglycéryl-2, les condensats d'acide adipique et de glycérol, pour lesquels une partie des groupes hydroxyle des glycérols ont réagi avec un mélange d'acides gras, tels que l'acide stéarique, l'acide caprique, l'acide stéarique et isostéarique et l'acide 12-hydroxystéarique, à l'image, notamment, de ceux commercialisé sous la marque Softisan 649 par la société Sasol ou tel que le polyacyladipate-2 de bis diglycéryle ; le propionate d'arachidyle commercialisé sous la marque Waxenol 801 par Alzo ; les esters de phytostérol ; les triglycérides d'acides gras et leurs dérivés, tels que les coco-glycérides hydrogénés ; les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$ ; les esters aliphatiques d'ester résultant de l'estérification d'un ester d'acide hydroxycarboxylique aliphatique par un acide carboxylique aliphatique ; les polyesters résultant de l'estérification, par un acide polycarboxylique, d'un ester d'acide hydroxy carboxylique aliphatique, ledit ester comprenant au moins deux groupes hydroxyle, tels que les produits Risocast DA-H®, et Risocast DA-C® ; et leurs mélanges.

Le ou les composés pâteux peuvent être présents dans une composition de l'invention en une teneur allant

de 0,1 à 30 % en poids d'agents de préférence encore de 0,5 à 20 % en poids, par rapport au poids total de la composition.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

Une composition selon l'invention peut, notamment, se présenter sous la forme d'une composition de maquillage et/ou de soin de la peau, en particulier un fond de teint.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'invention.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (fond de teint), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple

dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

La composition de l'invention peut se présenter sous la forme d'un produit de soin ou de préférence de maquillage, en particulier coloré, de la peau, plus spécifiquement du visage comme un fond de teint, un fard à joues ou à paupières, un fard à joues ou à paupières, un produit anti-cernes, un blush, une poudre libre ou compactée, ou encore un produit de maquillage du corps comme un produit de tatouage semi-permanent.

La composition selon l'invention peut être fabriquée par les procédés connus, généralement utilisés dans le domaine cosmétique.

La présente invention concerne également un procédé, plus spécifiquement cosmétique, de soin ou en particulier de maquillage de la peau et/ou des phanères (en particulier cheveux ou ongles), comprenant l'application de la composition selon l'invention décrite ci-dessus sur la peau et/ou ses phanères.

Les exemples qui suivent ont pour but d'illustrer de manière non limitative l'objet de la présente invention. Dans la demande, les teneurs, sauf mention express contraire, sont exprimées en poids par rapport au poids total de la composition.

## EXEMPLES

### Exemple 1 : préparation de la résine $MQT^{Pr}$

On utilise les résines suivantes :

Résine MQ = une résine MQ de formule $M_{0,43}Q_{0,57}$ et de $M_n$ = 3 230 dissoute dans du xylène à 70,8 % en poids de solides. La résine MQ a été fabriquée selon les techniques décrites pas Daudt dans le brevet US 2 676 182.

Résine de propyle T = une résine de propyle silsesquioxane à 74,8 % en poids dans du toluène. La résine de propyle silsesquioxane a été obtenue par hydrolyse de propyle trichlorosilane.

Une résine MQ, une résine de propyle T, du xylène et du KOH 1 M dans de l'eau dans les proportions présentées dans le tableau 1 sont introduits dans un tricol muni d'un agitateur, d'une sonde de température et d'un appareil de Dean Stark équipé d'un condenseur au sommet. Du xylène est pré-introduit dans l'appareil de Dean Stark afin de s'assurer de maintenir un niveau de solides de 50 % dans le réacteur. Le mélange dans le réacteur est maintenu à une température de reflux (entre 100 et 140 °C) pendant au moins 3 heures. Toute eau se formant dans le mélange réactionnel est éliminée en continu le cas échéant et piégée sous forme d'un azéotrope dans l'appareil de Dean Stark. Après 3 heures de reflux, l'eau est éliminée de l'appareil et le chauffage est poursuivi pendant 30 minutes supplémentaires. Après refroidissement du mélange, un excès d'acide acétique est ajouté afin de neutraliser le KOH dans le mélange. Le mélange est ensuite filtré afin d'éliminer les sels formés en le passant au travers d'un filtre sous pression. Un échange de solvant est réalisé en chauffant le mélange dans un évaporateur rotatif sous vide. Après élimination de la majorité du xylène, du décaméthylcyclopentasiloxane ou de l'isododécane est ajouté tout en continuant d'éliminer tout solvant aromatique résiduel. Les structures des résines de siloxane résultantes sont caractérisées par spectroscopie RMN $^{29}Si$ et CPG et les résultats sont récapitulés dans le tableau 2 ci-dessous.

**Tableau 1**

| Exemple # | Rapport massique de résines $MQ/T^{Pr}$ ajoutées | % en poids de résine MQ | % en poids de résine de propyle T | % en poids de xylène | % en poids de KOH 1 M | % en poids d'acide acétique |
|---|---|---|---|---|---|---|
| 1-a | (85:15) | 59,4 | 10,5 | 29,1 | 0,9 | 0,2 |
| 1-b | (50:50) | 34,9 | 34,8 | 29,1 | 0,9 | 0,2 |
| 1-c | (30:70) | 20,9 | 48,8 | 29,2 | 0,9 | 0,2 |
| 1-d | (95:5) | 67,1 | 3,5 | 28,3 | 0,9 | 0,2 |
| 1-e | (100:0) | 69,3 | 0 | 28,8 | 0,9 | 0,2 |

**Tableau 2**

| Exemple # | Structure de la résine selon la caractérisation RMN | % en poids de OH | Mn | Mw | Mw/Mn |
|---|---|---|---|---|---|
| Résine M Q | $m^{0,43}Q^{0,57}$ | | 3230 | 1 516 | 4,7 |
| Résine de propyle T | $T^{Pr}{}_{1,0}$ | 7,0 | 3470 | 11 400 | 3,3 |
| 1-a | $M_{0,374}Q_{0,529}:T^{Pr}{}_{0,097}$ | 1,4 | 5 880 | 271 000 | 46,1 |
| 1-b | $M_{0,248}Q_{0,341}:T^{Pr}{}_{0,412}$ | 2,1 | 6 640 | 3 860 000 | 581,3 |
| 1-c | $M_{0,162}Q_{0,217}:T^{PR}{}_{0,621}$ | 1,5 | 7 600 | 25 300 000 | 3329 |
| 1-d | $M_{0,419}Q_{0,5485}:T^{Pr}{}_{0,03}$ | 1,5 | | | |
| 1-e | MQ | 1,7 | 5 200 | 28 900 | 5,6 |

**Exemples 2-5 : Emulsion E/H**

Selon un mode particulier de l'invention, on utilise l'exemple 1-c décrit dans l'exemple 1 ci-dessus.

| | | Exemple 2 %massique (comparatif) | Exemple 3 %massique (selon l'invention) | Exemple 4 %massique (selon l'invention) | Exemple 5 %massique (comparatif) |
|---|---|---|---|---|---|
| A1 | Bis-PEG/PPG-14/14 Dimethicone & Cyclopentasiloxane (Abil EM97 de Goldschmidt) | 1,8 | 1,8 | 1,8 | 1,8 |
| | Isostearyl Diglyceryl Succinate (Inwitor 780K de Sasol) | 0,6 | 0,6 | 0,6 | 0,6 |
| | PEG-10 Dimethicone | 0,5 | 0,5 | 0,5 | 0,5 |
| | Isododecane | 13,32 | 13,32 | 13,32 | 13,32 |
| | Disteardimonium Hectorite & Propylene Carbonate (bentone gel ISD V de Elementis) | 5 | 5 | 5 | 5 |
| | DICAPRYLYL CARBONATE (Cetiol CC de Cognis) | 6 | 6 | 6 | 6 |
| | résine MQ / TPropyl 30 : 70 telle que préparée selon l'exemple 1-c décrit ci-dessus dans l'Isododécane | 11,38 | 11,38 | 11,38 | 11,38 |

(suite)

| | | Exemple 2 %massique (comparatif) | Exemple 3 %massique (selon l'invention) | Exemple 4 %massique (selon l'invention) | Exemple 5 %massique (comparatif) |
|---|---|---|---|---|---|
| A2 | Decamethyl cyclopentasiloxane | 3,5 | 3,5 | 3,5 | 3,5 |
| | CI 77492 & Disodium Stearoyl Glutamate & Aluminium Hvdroxide (1) | 1,43 | 1,43 | 1,43 | 1,43 |
| | CI 77491 & Disodium Stearoyl Glutamate & Aluminium Hydroxide (2) | 0,46 | 0,46 | 0,46 | 0,46 |
| | CI 77499 & Disodium Stearoyl Glutamate & Aluminium Hydroxide (3) | 0,22 | 0,22 | 0,22 | 0,22 |
| | CI 77891 & Disodium Stearoyl Glutamate & Aluminium Hydroxide (4) | 7,89 | 7,89 | 7,89 | 7,89 |
| A3 | Talc | - | 4 | 2 | - |
| | Nylon-12 | 8 | 4 | 4 | 4 |
| | SILICA (and) METHICONE (SB700 de Miyoshi Kasei) | - | - | 2 | - |
| | DIPHENYL DIMETHICONE/ VINYL DIPHENYL DIMETHICONE/ SILSESQUIOXANE CROSSPOLYMER (KSP300 de Shin Etsu) | - | - | - | 4 |
| B1 | Eau déminéralisée | QSP | QSP | QSP | QSP |
| | Magnesium Sulfate | 0,7 | 0,7 | 0,7 | 0,7 |
| | conservateurs | 0,3 | 0,3 | 0,3 | 0,3 |
| | TOTAL | 100% | 100% | 100% | 100% |

(1) 96.5% CI 77492 & 3.0% Disodium Stearoyl Glutamate & 0.5% Aluminium Hydroxide commercialisé sous la dénomination NAI-C33-9001-10 par la société MIYOSHI KASEI
(2) 96.5% CI 77491 & 3.0% Disodium Stearoyl Glutamate & 0.5% Aluminium Hydroxide commercialisé sous la dénomination NAI-C33-8001-10 par la société MIYOSHI KASEI
(3) 96.5% CI 77499 & 3.0% Disodium Stearoyl Glutamate & 0.5% Aluminium Hydroxide commercialisé sous la dénomination NAI-C33-7001-10 par la société MIYOSHI KASEI
(4) 97% CI 77891 & 2.5% Disodium Stearoyl Glutamate & 0.5% Aluminium Hydroxide commercialisé sous la dénomination NAI-TAO-77891 par la société MIYOSHI KASEI

Les exemples 3 et 4 correspondent à des compositions selon l'invention.

Les exemples 2 et 5 sont des exemples comparatifs.

Mode opératoire

On pèse les constituants de la phase A2. Le mélange est passé sur broyeur tri-cylindre.
On pèse ensuite les constituants de la phase A1 dans le bêcher principal et on le place au bain-marie (75-80°C). Lorsque le mélange est homogène, on refroidit le mélange jusqu'à température ambiante.
On incorpore A2 à la phase A1, sous agitation au Moritz à 1500 tours/min.
Puis on ajoute successivement les constituants de la phase A3, en gardant la même agitation.
On pèse les constituants de la phase B. On porte à ébullition la phase B, jusqu'à dissolution complète des constituants. On refroidit la phase B jusqu'à 50°C.
La phase B est ensuite ajoutée en filet dans la phase A1+A2+A3, sous agitation au Moritz à 3200 tr/min.

Protocole de mesures instrumentales de la matité immédiate et tenue de la matité

La matité et la tenue de la matité peuvent être mesurées au moyen du protocole décrit ci-après. La matité d'une région de la peau, par exemple, du visage est mesurée à l'aide d'une caméra polarimétrique, qui est un système d'imagerie polarimétrique en noir et blanc, avec laquelle des images en lumière polarisée parallèle (P) et croisée (C) sont acquises.
Par analyse de l'image résultant de la soustraction des deux images (P-C), la brillance est quantifiée en mesurant le niveau de gris moyen des 5 % de pixels les plus brillants correspondant aux zones de brillance.
Plus précisément, les mesures sont effectuées sur un panel de personnes, qui sont gardées en salle d'attente climatisée (22 °C +/- 2 °C) 15 mn avant le début du test. Elles se démaquillent et une image d'une de leurs joues est acquise avec la caméra polarimétrique. Cette image permet de mesurer la brillance à T0 avant maquillage. Puis environ 100 mg de chaque composition cosmétique telle que préparée ci-dessus sont pesés dans un verre de montre, et sont appliqués aux doigts nus sur le demi visage sur lequel la mesure à T0 a été réalisée. Après un temps de séchage de 15 mn, une image de la joue maquillée est acquise avec la caméra polarimétrique. Cette image permet de mesurer la brillance juste après maquillage (Timm). Les modèles retournent alors en salle climatisée pendant 3 h.
Enfin, une image de la joue maquillée après 3h d'attente est acquise avec la caméra polarimétrique. Cette image permet de mesurer la brillance après 3 h de maquillage (T3h). Les résultats sont exprimés en calculant la différence (Timm - T0) qui mesure l'effet du maquillage. Une valeur négative signifie que le maquillage diminue la brillance de la peau et qu'il est donc matifiant.
La différence (T3h - Timm) mesurant la tenue de cet effet est ensuite calculée. La valeur obtenue doit être la plus faible possible ce qui signifie que la matité du maquillage ne change pas au cours de temps.
Pour les mesures effectuées, on considère que :

+ effet léger ou tenue faible
++ effet moyen ou tenue moyenne
+++ effet important ou tenue bonne
++++ effet très important ou tenue très bonne

Protocole de mesures instrumentales de la couleur immédiate et tenue de la couleur

On utilise le même protocole que précédemment pour la matité mais au lieu de mesurer la brillance, on effectue une mesure colorimétrique de la peau avant et après maquillage en mesurant les indices a*,b* et L* à savoir les indices rouge, jaune et la luminance.
Pour chaque femme, une image des joues est prise à T0 (avant maquillage), à Timm (15 minutes après maquillage) à T3h (3h après maquillage), à l'aide d'une chromasphère, de définition 410x410 pixels.
Chaque image obtenue à l'aide de la caméra est exploité en couleur. La couleur est quantifiée par les indices de rouge et de jaune, la luminance, l'écart de couleur (respectivement a*,b*L et deltaE).
Le delta E, dE ou encore ΔE, est défini comme une mesure de différence entre deux couleurs. Voici la formule établie en 1976 :

$$\Delta E^* = \sqrt{((L_1 - L_2)^2 + (a_1 - a_2)^2 + (b_1 - b_2)^2}$$

où :

$L_1,a_1,b_1$ sont les coordonnées dans l'espace colorimétrique de la première couleur à comparer et $L_2,a_2,b_2$ celles de la seconde.

Résultats

En termes de tenue des propriétés cosmétiques. Les exemples 2, 3, 4 et 5 présentent une bonne tenue des propriétés cosmétiques (matité et couleur) :

(T3h - Timm) notation +++

En revanche, des disparités sont observées en termes de confort à l'application et de résultat maquillage.
En effet, une évaluation sensorielle desdites compositions a été réalisée sur un panel de personnes utilisatrices de fond de teint. Après application libre de chaque composition sur le visage, chaque personne évalue la perception de ladite composition au moment de l'application et en termes de résultat maquillage. Pour les exemples 3 et 4, selon l'invention, l'application est facile sans effet « crissant » sur la peau. Le produit glisse parfaitement sous les doigts, ce qui rend l'application agréable. Le fini maquillage est doux au touché, non collant.
A l'inverse, lorsque l'on applique les formules 2 et 5, exemples comparatifs, on ressent un frottement sur la peau, qui rend l'application inconfortable et gênante. Le fini maquillage est beaucoup moins doux au toucher.
Ces résultats montrent que les charges de nature minérale, telles que le talc et la silice, permettent, en association avec les résines de siloxane selon l'invention, de conférer une bonne tenue des propriétés cosmétiques (matité et couleur), tout en donnant à la composition de bonnes propriétés de confort à l'application (toucher doux, non collant), comparativement aux charges organiques telles que les charges organiques de type polyamide (nylon-12) ou poudres d'élastomère de siloxane (KSP300). Ces dernières peuvent être utilisées comme charges additionnelles, mais nécessairement en association avec au moins une charge minérale en quantité majoritaire ou au moins égale à la somme des charges organiques selon l'invention.

**Exemple 6 : poudre compacte**

| | | %massique |
|---|---|---|
| **A1** | Dioxyde de titane CI 77891 | 2 |
| | Oxyde de fer jaune CI 77492 | 0,92 |
| | Oxyde de fer rouge CI 77491 | 0,64 |
| | Oxyde de fer noir CI 77499 | 0,1 |
| | **Mica** | 10 |
| **A2** | **STEARATE DE MAGNESIUM** | 2,51 |
| | **MICA** | 20 |
| | **POUDRE DE NYLON 12** | 20 |
| | **TALC** | 34 |
| **B1** | résine MQ / TPropyl 30 : 70 telle que préparée selon l'exemple 1-c décrit ci-dessus dans l'Isododécane | 8 |
| | POLY DIMETHYLSILOXANE (VISCOSITE: 10 CST) (FLUID DC 200 10 CST de Dow corning) | 1,23 |
| | CAPRYLIC/CAPRIC TRIGLYCERIDE (MYRITOL 318® par la société COGNIS) | 4 |
| **B2** | conservateurs | 0,6 |
| | TOTAL | 100% |

Mode opératoire

On pèse les constituants de la phase A1.
On pèse les constituants de la phase A2. Les phases A1 et A2 sont ajoutées dans un mélangeur type Lodige, pendant

10 minutes.
On ajoute les constituants de la phase B1 successivement puis on homogénéise dans le mélangeur Lodige, pendant 10 minutes.
On ajoute enfin la phase B2 et on homogénéise au mélangeur 3 minutes.
La composition est tamisée à l'aide d'un tamis 250µm.
La composition est enfin passée au broyeur type Alpine.

**Exemple N° 7 : Poudre de teint :**

| Exemple n°6 | | |
|---|---|---|
| **A** | Pigments | 10 |
| | Talc | 64,7 |
| | Poudre de Nylon | 10 |
| | Conservateur | 0,3 |
| **B** | Huile de Parleam | 8 |
| | Résine telle que décrite dans WO 2005/075542* | 10 |
| * résine MQ / TPropyl 30 : 70 telle que préparée selon l'exemple 1-c décrit ci-dessus dans l'Isododécane | | |

Mode opératoire

- On pèse les matières premières de la phase A et on homogénéise par un mélangeur pendant 10 minutes.
- On pèse les matières premières de la phase B, que l'on ajoute à la phase A.
- On homogénéise le mélange au mélangeur pendant 3 minutes.
- On passe le mélange A+B au broyeur Alpine.

**Revendications**

1. Procédé cosmétique de maquillage et/ou de soin de la peau, comprenant l'application sur la peau d'une composition comprenant, dans un milieu physiologiquement acceptable :

   - i) une résine de siloxane comprenant les unités :

     (i) $(R^1{}_3SiO_{1/2})a$
     (ii) $(R^2{}_2SiO_{2/2})b$
     (iii) $(R^3SiO_{3/2})c$ et
     (iv) $(SiO_{4/2})d$

   avec

     $R^1$, $R^2$ et $R^3$ représentant indépendamment un groupement alkyle ayant de 1 à 8 atomes de carbone, un groupement aryle, un groupement carbinol ou un groupement amino,
     a étant compris entre 0,05 et 0,5,
     b étant compris entre zéro et 0,3,
     c étant supérieur à zéro,
     d étant compris entre 0,05 et 0,6,
     a + b + c + d = 1,

   à condition que plus de 40 % en moles des groupements $R^3$ de la résine de siloxane soient des groupements

propyle, et

- ii) au moins une charge minérale choisie parmi le talc, le mica, la silice, le kaolin, le nitrure de bore, le carbonate de calcium précipité, le carbonate et l'hydro-carbonate de magnésium, l'hydroxyapatite, les microsphères de silice creuses, les microcapsules de verre ou de céramique, l'argile, le quartz, la poudre de diamant naturel et leur mélange.

2. Procédé cosmétique de maquillage et/ou de soin des matières kératiniques selon la revendication 1, **caractérisé en ce que** la résine de siloxane présente dans ladite composition comprend les unités :

(i) $(R^1{}_3SiO_{1/2})a$
(iii) $(R^3SiO_{3/2})c$ et
(iv) $(SiO_{4/2})d$

avec

$R^1$ et $R^3$ représentant indépendamment un groupement alkyle ayant de 1 à 8 atomes de carbone, de préférence $R^1$ est un groupe méthyle et $R^3$ est un groupe propyle,
a étant compris entre 0,05 et 0,5,
c étant supérieur à zéro,
d étant compris entre 0,05 et 0,6,
a + c + d = 1,

à condition que plus de 40 % en moles des groupements $R^3$ de la résine de siloxane soient des groupements propyle.

3. Procédé selon l'une quelconque des revendications 1 à 2, **caractérisé en ce que** ladite résine de siloxane est obtenue par un procédé comprenant la réaction entre :

A) une résine MQ comprenant au moins 80 % en moles d'unités $(R^1{}_3SiO_{1/2})_a$ et $(SiO_{4/2})_d$

$R^1$ représentant un groupement méthyle,
a et d étant supérieurs à zéro,
le rapport a/d étant compris entre 0,5 et 1,5 ;

et
B) une résine de T propyle comprenant au moins 80 % en moles d'unités $(R^3SiO_{3/2})c$,

$R^3$ représentant un groupement propyle,
c étant supérieur à zéro,

où le ratio massique A/B est compris entre 95:5 et 15:85, de préférence le ratio massique A/B est de 30 :70.

4. Procédé selon l'une des revendications précédentes, **caractérisé en ce que** ladite composition comprend une quantité de résine de siloxane en poids de matière active (extrait sec), allant de 0,5 à 60% en poids, par rapport au poids total de la composition, de préférence de 3 à 60 % en poids, et mieux de 4 à 60% en poids par rapport au poids total de ladite composition.

5. Procédé selon l'une quelconque des revendications précédentes, **caractérisé en ce que** ladite composition se présente sous la forme de poudres, de dispersion anhydre ou d'émulsions, en particulier d'émulsions inverses.

6. Procédé selon l'une quelconque des revendications précédentes, **caractérisé en ce que** la charge minérale présente dans ladite composition est choisie parmi le talc et la silice.

7. Procédé selon l'une quelconque des revendications précédentes, **caractérisé en ce que** ladite composition comprend en outre au moins une autre charge minérale ou organique.

8. Procédé selon la revendication précédente, **caractérisé en ce que** ladite autre charge est minérale, et est éventuellement associée à au moins une charge organique.

**9.** Procédé cosmétique de maquillage et/ou de soin de la peau selon la revendication 7 ou 8, **caractérisé en ce que** ladite charge organique est choisie parmi les poudres de polyamide, les poudres de polymères acryliques, notamment les poudres de polyméthyl méthacrylate, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, les poudres de cellulose, de poly-β-alanine et de polyéthylène, les poudres de polymères de tétrafluoroéthylène, la lauroyl-lysine, l'amidon, les microsphères creuses polymériques telles que celles de chlorure de polyvinylidène/acrylonitrile, de copolymères d'acide acrylique, les microbilles de résine de silicone, les particules de polyorganosiloxanes élastomères, notamment obtenues par polymérisation d'organopolysiloxane ayant au moins deux atomes d'hydrogène liés chacun à un atome de silicium et d'un organopolysiloxane comprenant au moins deux groupes à insaturation éthylénique en présence de catalyseur platine, ou encore les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

**10.** Procédé selon la revendication précédente, **caractérisé en ce que** la charge organique correspond aux poudres de polyamide.

**11.** Procédé selon l'une quelconque des revendications précédentes, **caractérisé en ce que** la ou les charge(s) est ou sont présente(s) dans la composition en une teneur allant de 0,01 à 50% en poids, notamment de 0,01 % à 30 % en poids, par rapport au poids total de la composition, de préférence allant de 0,01 % à 20 % en poids.

**12.** Composition de maquillage et/ou de soin de la peau susceptible d'être mise en oeuvre dans un procédé selon l'une des revendications précédentes, comprenant dans un milieu physiologiquement acceptable,

- i) au moins une résine de siloxane comprenant les unités :

(i) $(R^1{}_3SiO_{1/2})a$
(ii) $(R^2{}_2SiO_{2/2})b$
(iii) $(R^3SiO_{3/2})c$ et
(iv) $(SiO_{4/2})d$

avec

$R^1$, $R^2$ et $R^3$ représentant indépendamment un groupement alkyle ayant de 1 à 8 atomes de carbone, un groupement aryle, un groupement carbinol ou un groupement amino,
a étant compris entre 0,05 et 0,5,
b étant compris entre zéro et 0,3,
c étant supérieur à zéro,
d étant compris entre 0,05 et 0,6,
a+b+c+d=1,

à condition que plus de 40 % en moles des groupements $R^3$ de la résine de siloxane soient des groupements propyle, et
- ii) au moins une charge minérale telle que définie dans la revendication 1 ou 6.

**13.** Ensemble cosmétique comprenant :

a. un récipient délimitant au moins un compartiment, ledit récipient étant fermé par un élément de fermeture ; et
b. une composition disposée à l'intérieur dudit compartiment, la composition étant définie selon la revendication précédente.

**14.** Ensemble cosmétique tel que défini dans la revendication 13 comprenant en outre un applicateur sous forme d'un bloc de mousse ou d'élastomère, d'un feutre, ou d'une spatule.

## Claims

**1.** A cosmetic method for making up and/or caring for the skin, comprising the application to the skin of a composition comprising, in a physiologically acceptable medium:

- i) a siloxane resin comprising the units:

(i) $(R^1{}_3SiO_{1/2})a$
(ii) $(R^2{}_2SiO_{2/2})_b$
(iii) $(R^3SiO_{3/2})_c$ and
(iv) $(SiO_{4/2})_d$

with

$R^1$, $R^2$ and $R^3$ independently representing an alkyl group having from 1 to 8 carbon atoms, an aryl group, a carbinol group or an amino group,
a being between 0.05 and 0.5,
b being between zero and 0.3,
c being greater than zero,
d being between 0.05 and 0.6,
a+b+c+d=1,

provided that more than 40 mol% of the $R^3$ groups of the siloxane resin are propyl groups, and
- ii) at least one inorganic filler selected from talc, mica, silica, kaolin, boron nitride, precipitated calcium carbonate, magnesium carbonate, basic magnesium carbonate, hydroxyapatite, hollow silica microspheres, glass or ceramic microcapsules, clay, quartz, natural diamond powder and their mixture.

2. The cosmetic method for making up and/or caring for keratinous substances according to claim 1, **characterized in that** the siloxane resin present in said composition comprises the units:

(i) $(R^1{}_3SiO_{1/2})_a$
(iii) $(R^3SiO_{3/2})_c$ and
(iv) $(siO_{4/2})_d$

with

$R^1$ and $R^3$ independently representing an alkyl group having from 1 to 8 carbon atoms; preferably, $R^1$ is a methyl group and $R^3$ is a propyl group,
a being between 0.05 and 0.5,
c being greater than zero,
d being between 0.05 and 0.6,
a + c + d = 1,

provided that more than 40 mol% of the $R^3$ groups of the siloxane resin are propyl groups.

3. The method according to anyone of claims 1 and 2, **characterized in that** said siloxane resin is obtained by a process comprising the reaction between:

A) an MQ resin comprising at least 80 mol% of $(R^1{}_3SiO_{1/2})a$ and $(SiO_{4/2})_d$ units,

$R^1$ representing a methyl group,
a and d being greater than zero,
the ratio a/d being between 0.5 and 1.5;

and
B) a propyl T resin comprising at least 80 mol% of $(R^3SiO_{3/2})_c$ units,

$R^3$ representing a propyl group,
c being greater than zero,

where the A/B ratio by weight is between 95:5 and 15:85; preferably, the A/B ratio by weight is 30:70.

4. The method according to anyone of the preceding claims, **characterized in that** said composition comprises an

amount of siloxane resin, by weight of active material (on a dry basis), ranging from 0.5 to 60% by weight, with respect to the total weight of the composition, preferably from 3 to 60% by weight and better still from 4 to 60% by weight, with respect to the total weight of said composition.

**5.** The method according to anyone of the preceding claims, **characterized in that** said composition is provided in the form of powders, of anhydrous dispersions or of emulsions, in particular of inverse emulsions.

**6.** The method according to anyone of the preceding claims, **characterized in that** the inorganic filler present in said composition is chosen from talc and silica.

**7.** The method according to anyone of the preceding claims, **characterized in that** said composition additionally comprises at least one other inorganic or organic filler.

**8.** The method according to the preceding claim, **characterized in that** said other filler is inorganic and is optionally used in combination with at least one organic filler.

**9.** The cosmetic method for making up and/or caring for the skin according to claim 7 or 8, **characterized in that** said organic filler is chosen from powders formed of polyamide, powders formed of acrylic polymers, in particular powders formed of polymethyl methacrylate, of poly(methyl methacrylate/ethylene glycol dimethacrylate), of poly(allyl methacrylate/ethylene glycol dimethacrylate) or of ethylene glycol dimethacrylate/lauryl methacrylate copolymer, powders formed of cellulose, of poly-β-alanine and of polyethylene, powders formed of tetrafluoroethylene polymers, lauroyl lysine, starch, hollow polymeric microspheres, such as those of poly(vinylidene chloride/acrylonitrile), or of acrylic acid copolymers, silicone resin microbeads, polyorganosiloxane elastomer particles, in particular obtained by polymerization of organopolysiloxane having at least two hydrogen atoms each bonded to a silicon atom and of an organopolysiloxane comprising at least two groups possessing ethylenic unsaturation, in the presence of a platinum catalyst, or else metal soaps derived from organic carboxylic acids having from 8 to 22 carbon atoms, preferably from 12 to 18 carbon atoms, for example zinc stearate, magnesium stearate, lithium stearate, zinc laurate or magnesium myristate.

**10.** The method according to the preceding claim, **characterized in that** the organic filler corresponds to polyamide powders.

**11.** The method according to any one of the preceding claims, **characterized in that** the filler(s) is or are present in the composition in a content ranging from 0.01 to 50% by weight, in particular from 0.01 to 30% by weight, with respect to the total weight of the composition, and preferably ranging from 0.01 to 20% by weight.

**12.** A composition for making up and/or caring for the skin capable of being employed in a method as claimed in one of the preceding claims, comprising, in a physiologically acceptable medium:

- i) at least one siloxane resin comprising the units:

(i) $(R^1{}_3SiO_{1/2})_a$
(ii) $(R^2{}_2SiO_{2/2})_b$
(iii) $(R^3SiO_{3/2})_c$ and
(iv) $(siO_{4/2})_d$

with

$R^1$, $R^2$ and $R^3$ independently representing an alkyl group having from 1 to 8 carbon atoms, an aryl group, a carbinol group or an amino group,
a being between 0.05 and 0.5,
b being between zero and 0.3,
c being greater than zero,
d being between 0.05 and 0.6,
a+b+c+d=1,

provided that more than 40 mol% of the $R^3$ groups of the siloxane resin are propyl groups, and
- ii) at least one inorganic filler as defined in either of claims 1 and 6.

**13.** A cosmetic combination comprising:

a. a container delimiting at least one compartment, said container being closed by a closing element; and
b. a composition positioned inside said compartment, the composition being defined as claimed in the preceding claim.

**14.** The cosmetic combination according to claim 13, additionally comprising an applicator in the form of a pad of foam or elastomer, of a felt-tipped pen or of a spatula.

**Patentansprüche**

**1.** Kosmetisches Verfahren zum Schminken und/oder zur Pflege der Haut, umfassend die Applikation einer Zusammensetzung auf die Haut, umfassend in einem physiologisch verträglichen Milieu:

- i) ein Siloxanharz, umfassend die Einheiten:

(i) $(R^1{}_3SiO_{1/2})a$
(ii) $(R^2{}_2SiO_{2/2})b$
(iii) $(R^3SiO_{3/2})c$ und
(iv) $(SiO_{4/2})d$

wobei

$R^1$, $R^2$ und $R^3$ unabhängig voneinander für eine Alkylgruppe mit 1 bis 8 Kohlenstoffatomen, eine Arylgruppe, eine Carbinolgruppe oder eine Aminogruppe stehen, a zwischen 0,05 und 0,5 liegt,
b zwischen 0 und 0,3 liegt,
c größer als 0 ist,
d zwischen 0,05 und 0,6 liegt,
a+b+c+d=1,

unter der Bedingung, dass mehr als 40 Mol-% der $R^3$-Gruppen des Siloxanharzes Propylgruppen sind, und
- ii) wenigstens einen mineralischen Füllstoff, ausgewählt aus Talkum, Glimmer, Siliciumdioxid, Kaolin, Bornitrid, gefälltes Calciumcarbonat, Magnesiumcarbonat und -hydrogencarbonat, Hydroxyapatit, Siliciumdioxid- Mikrohohlkugeln, Keramik- oder Glasmikrokapseln, Ton, Quarz, natürlichem Diamentpulver und ihrer Mischung.

**2.** Kosmetisches Verfahren zum Schminken und/oder zur Pflege von Keratinmaterialien gemäß Anspruch 1, **dadurch gekennzeichnet, dass** das Siloxanharz in besagter Zusammensetzung die Einheiten aufweist:

(i) $(R^1{}_3SiO_{1/2})a$
(iii) $(R^3SiO_{3/2})c$ und
(iv) $(SiO_{4/2})d$

wobei

$R^1$ und $R^3$ unabhängig voneinander für eine Alkylgruppe mit 1 bis 8 Kohlenstoffatomen stehen, vorzugsweise $R^1$ eine Methylgruppe ist und $R^3$ eine Propylgruppe ist,
a zwischen 0,05 und 0,5 liegt,
c größer als 0 ist,
d zwischen 0,05 und 0,6 liegt,
a+c+d=1,

unter der Bedingung, dass mehr als 40 Mol-% der $R^3$-Gruppen des Siloxanharzes Propylgruppen sind.

**3.** Verfahren gemäß einem der Ansprüche 1 bis 2, **dadurch gekennzeichnet, dass** besagtes Siloxanharz mithilfe eines Verfahrens erhalten wird, umfassend die Reaktion von:

A) einem MQ-Harz, umfassend wenigstens 80 Mol-% an $(R^1{}_3SiO_{1/2})_a$- und $(SiO_{4/2})_d$-Einheiten,

wobei $R^1$ für eine Methylgruppe steht,
a und d größer als 0 sind,
das Verhältnis a/d zwischen 0,5 und 1,5 beträgt;

B) einem Propyl-T-Harz, umfassend wenigstens 80 Mol-% an $(R^3SiO_{3/2})$c-Einheiten,

wobei $R^3$ für eine Propylgruppe steht,
c größer als 0 ist,

wobei das Massenverhältnis A/B zwischen 95:5 und 15:85 beträgt, vorzugsweise das Massenverhältnis A/B 30:70 beträgt.

**4.** Verfahren gemäß einem der vorhergehenden Ansprüche, **dadurch gekennzeichnet, dass** besagte Zusammensetzung eine Gewichtsmenge an Siloxanharz als Wirkstoff (Trockensubstanz) umfasst, die 0,5 bis 60 Gew.-% bezogen auf das Gesamtgewicht der Zusammensetzung, bevorzugt 3 bis 60 Gew.-% und bevorzugter 4 bis 60 Gew.-% bezogen auf das Gesamtgewicht besagter Zusammensetzung beträgt.

**5.** Verfahren gemäß einem der vorhergehenden Ansprüche, **dadurch gekennzeichnet, dass** besagte Zusammensetzung in Form von Pulvern, einer wasserfreien Dispersion oder von Emulsionen, insbesondere von inversen Emulsionen, vorliegt.

**6.** Verfahren gemäß einem der vorhergehenden Ansprüche, **dadurch gekennzeichnet, dass** der in besagter Zusammensetzung enthaltene mineralische Füllstoff aus Talkum und Siliciumdioxid ausgewählt ist.

**7.** Verfahren gemäß einem der vorhergehenden Ansprüche, **dadurch gekennzeichnet, dass** besagte Zusammensetzung außerdem wenigstens einen weiteren mineralischen oder organischen Füllstoff umfasst.

**8.** Verfahren gemäß dem vorhergehenden Anspruch, **dadurch gekennzeichnet, dass** besagter weiterer Füllstoff ein mineralischer Füllstoff ist und möglicherweise mit wenigstens einem organischen Füllstoff assoziiert ist.

**9.** Kosmetisches Verfahren zum Schminken und/oder zur Pflege der Haut gemäß Anspruch 7 oder 8, **dadurch gekennzeichnet, dass** besagter organischer Füllstoff ausgewählt ist aus Polyamidpulvern, Pulvern von Acrylpolymeren, insbesondere Polymethylmethacrylat-, Polyethylenglykolmethylmethacrylat/-dimethylacrylat-, Polyethylenglykolallylmethacrylat/- dimethacrylatpulvern, Ethylenglykoldimethacrylat/Laurylmethacrylat-Copolymerpulvern, Cellulose-, Poly-β-alanin- und Polyethylenpulvern, Polytetrafluorethylenpulvern, Lauroyl-Lysin, Stärke, Polymermikrohohlkugeln, wie solche aus Polyvinyliden/Acrylnitrilchlorid, aus Acrylsäure-Copolymeren, Siliconharz-Kügelchen, Elastomer-Polyorganosiloxan-Partikeln, insbesondere die durch Polymerisation von Organopolysiloxan, das wenigstens zwei Wasserstoffatome besitzt, die jeweils an ein Siliciumatom gebunden sind, und einem Organopolysiloxan, das wenigstens zwei ungesättigte Ethylengruppen umfasst, in Gegenwart eines Platinkatalysators erhalten werden, oder auch Metallseifen, die von organischen Carbonsäuren mit 8 bis 22 Kohlenstoffatomen, vorzugsweise 12 bis 18 Kohlenstoffatomen, abgeleitet sind, zum Beispiel Zink-, Magnesium oder Lithiumstearat, Zinklaureat, Magnesiummyristat.

**10.** Verfahren gemäß dem vorhergehenden Anspruch, **dadurch gekennzeichnet, dass** der organische Füllstoff Polyamidpulvern entspricht.

**11.** Verfahren gemäß einem der vorhergehenden Ansprüche, **dadurch gekennzeichnet, dass** der oder die Füllstoffe in der Zusammensetzung mit einem Gehalt von 0,01 bis 50 Gew.-%, insbesondere von 0,01 bis 30 Gew.-% bezogen auf das Gesamtgewicht der Zusammensetzung, vorzugsweise von 0,01 bis 20 Gew.-%, vorliegen.

**12.** Zusammensetzung zum Schminken und/oder zur Pflege der Haut, die in einem Verfahren gemäß einem der vorhergehenden Ansprüche verwendet werden kann, umfassend in einem physiologisch verträglichen Milieu:

- i) wenigstens ein Siloxanharz, umfassend die Einheiten:

(i) $(R^1{}_3SiO_{1/2})$a
(ii) $(R^2{}_2SiO_{2/2})$b
(iii) $(R^3SiO_{3/2})$c und

(iv) $(SiO_{4/2})d$

wobei

$R^1$, $R^2$ und $R^3$ unabhängig voneinander für eine Alkylgruppe mit 1 bis 8 Kohlenstoffatomen, eine Arylgruppe, eine Carbinolgruppe oder eine Aminogruppe stehen, a zwischen 0,05 und 0,5 liegt,
b zwischen 0 und 0,3 liegt,
c größer als 0 ist,
d zwischen 0,05 und 0,6 liegt,
a+b+c+d=1,

unter der Bedingung, dass mehr als 40 Mol-% der $R^3$-Gruppen des Siloxanharzes Propylgruppen sind, und
- ii) wenigstens einen wie in Anspruch 1 oder 6 definierten mineralischen Füllstoff.

**13.** Kosmetikset umfassend:

a. einen Behälter, der wenigstens eine Kammer begrenzt, wobei besagter Behälter mit einem Verschließelement verschlossen ist; und
b. eine Zusammensetzung, die im Innern besagter Kammer angeordnet ist, wobei die Zusammensetzung gemäß dem vorhergehenden Anspruch definiert ist.

**14.** Kosmetikset, wie in Anspruch 13 definiert, umfassend außerdem einen Applikator in Form eines Schaumstoff- oder Elastomerblocks, eines Filzes oder einer Spachtel.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2005075542 A
- US 2814601 A
- US 2857356 A
- WO 04055081 A
- FR 2232303 A
- US 5162410 A
- WO 2004073626 A
- EP 1411069 A
- WO 04028488 A
- WO 2007071706 A
- US 2007142575 A
- US 2007142599 A
- US 5236986 A
- US 5412004 A
- US 5837793 A
- US 5811487 A
- JP 61194009 A
- EP 242219 A
- EP 285886 A
- EP 765656 A
- US 5538793 A
- EP 1400234 A
- EP 708114 A
- WO 02056847 A
- WO 0247619 A
- US 5783657 A
- EP 1266647 A
- FR 0216039
- WO 03105788 A
- FR 2892303 A
- FR 2900819 A
- FR 28994476 A
- WO 02056854 A
- JP 5339125 A
- EP 486135 A
- JP H0586984 B
- FR 0450074
- FR 2844710 A
- EP 1201221 A
- US 2004175338 A
- EP 847752 A
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618
- US 2676182 A